(19) Europäisches Patentamt
European Patent Office
Office européen des brevets

(11) **EP 3 513 667 A1**

(12) **EUROPEAN PATENT APPLICATION**
published in accordance with Art. 153(4) EPC

(43) Date of publication:
24.07.2019 Bulletin 2019/30

(51) Int Cl.:
***A24F 47/00*** *(2006.01)*

(21) Application number: 17853070.5

(86) International application number:
PCT/JP2017/033889

(22) Date of filing: 20.09.2017

(87) International publication number:
WO 2018/056300 (29.03.2018 Gazette 2018/13)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: 26.09.2016 PCT/JP2016/078258

(71) Applicant: **Japan Tobacco Inc.**
**Tokyo 105-8422 (JP)**

(72) Inventor: **NAKANO, Takuma**
**Tokyo 130-8603 (JP)**

(74) Representative: **Isarpatent**
**Patent- und Rechtsanwälte Behnisch Barth Charles**
**Hassa Peckmann & Partner mbB**
**Friedrichstrasse 31**
**80801 München (DE)**

(54) **FLAVOR INHALER**

(57) The present invention controls the amounts of an inhaled aerosol and flavor independently from each other. A flavor inhaler is provided with: a mouthpiece; a first atomizing unit that atomizes an aerosol source and generates an aerosol; a flavor source that is provided between the first atomizing unit and the mouthpiece; a first flow path configured so as to guide the aerosol generated by the first atomizing unit through the flavor source to the mouthpiece; a second atomizing unit that atomizes an aerosol source and generates an aerosol; a second flow path configured so as to guide the aerosol generated by the second atomizing unit to the mouthpiece without said aerosol passing through the flavor source; and a control unit that makes it possible to change the generated aerosol amount in at least one of the first atomizing unit and the second atomizing unit.

Fig. 1

EP 3 513 667 A1

## Description

TECHNICAL FIELD

The present invention relates to a flavor inhaler which adds flavor to aerosol to be inhaled.

BACKGROUND ART

A type of flavor inhaler, by which flavor is inhaled without a burning process, has been known. For example, a flavor inhaler comprises an atomizing unit for atomizing an aerosol source without a burning process, and a flavor source arranged in a position closer to a mouthpiece side than a position of the atomizing unit (for example, refer to Patent Literature 1)

CITATION LIST

PATENT LITERATURE

PTL 1: PCT international publication No. WO 2015/179388

SUMMARY OF INVENTION

TECHNICAL PROBLEM

In a flavor inhaler disclosed in Patent Literature 1, it is not possible to control the quantity of aerosol to be inhaled and the quantity of flavor, which is originated from a flavor source, to be inhaled, independently from each other.

The present invention has been made by taking the above matter into consideration; and an object of the present invention is to provide a flavor inhaler which can control the quantity of aerosol to be inhaled and the quantity of flavor to be inhaled independently from each other.

SOLUTION TO PROBLEM

For solving the above problem, a mode of the present invention comprises a flavor inhaler which comprises: a mouthpiece; a first atomizing unit for atomizing an aerosol source for generating aerosol; a flavor source positioned between the first atomizing unit and the mouthpiece; a first flow path constructed for guiding the aerosol generated in the first atomizing unit to the mouthpiece through the flavor source; a second atomizing unit for atomizing an aerosol source for generating aerosol; a second flow path constructed for guiding the aerosol generated in the second atomizing unit to the mouthpiece without passing through the flavor source; and a control unit which can change the quantity of aerosol to be generated in at least one of the first atomizing unit and the second atomizing unit.

Another mode of the present invention comprises the flavor inhaler of the above mode, wherein the control unit controls the quantity of aerosol to be generated in the first atomizing unit and the quantity of aerosol to be generated in the second atomizing unit in an independent manner.

Another mode of the present invention comprises the flavor inhaler of the above mode, wherein the flavor source adds a quantity of flavor components, that corresponds to the quantity of aerosol generated in the first atomizing unit, to the aerosol passing through the flavor source, and the control unit controls the quantity of aerosol to be generated in the first atomizing unit in such a manner that a predetermined quantity of the flavor components is delivered to the mouthpiece.

Another mode of the present invention comprises the flavor inhaler of the above mode, wherein the control unit controls, in response to a set value for setting at least one of a total quantity of aerosol and a quantity of flavor components which should be delivered to the mouthpiece, the quantities of aerosol to be generated in the first atomizing unit and the second atomizing unit.

Another mode of the present invention comprises the flavor inhaler of the above mode, and further comprises a user setting unit for receiving a user instruction relating to the setting value.

Another mode of the present invention comprises the flavor inhaler of the above mode, wherein the user setting unit is able to communicate with an external device, and is constructed to receive the user instruction via a user interface screen displayed on a display unit in the external device.

Another mode of the present invention comprises the flavor inhaler of the above mode, wherein the user setting unit is constructed to receive the user instruction relating to both the total quantity of aerosol and the quantity of flavor components via a single user interface screen displayed on a display unit in the external device.

Another mode of the present invention comprises the flavor inhaler of the above mode, wherein the user setting unit is constructed to receive the user instruction relating a ratio between the total quantity of aerosol and the quantity of flavor components via the user interface screen.

Another mode of the present invention comprises the flavor inhaler of the above mode, wherein the control unit makes the user interface screen to give notice of an error, in the case that the quantity of aerosol to be generated, based on the user instruction given via the user interface screen, in at least one of the first atomizing unit and the second atomizing unit exceeds a predetermined maximum value.

Another mode of the present invention comprises the flavor inhaler of the above mode, wherein the control unit determines, based on a variable range of the quantity of aerosol to be generated in the first atomizing unit and a variable range of the quantity of aerosol to be generated in the second atomizing unit, a variable range of the total quantity of aerosol and a variable range of the quantity of flavor components, and supplies the values representing the variable range of the total quantity of aerosol and the variable range of the quantity of flavor components to the external device for displaying them in the user interface screen.

Another mode of the present invention comprises the flavor inhaler of the above mode, wherein the control unit stops generation of the aerosol in the first atomizing unit or the second atomizing unit, in the case that the user instruction given via the user interface screen satisfies a predetermined condition.

Another mode of the present invention comprises the flavor inhaler of the above mode, wherein the first atomizing unit and the second atomizing unit are constructed to atomize aerosol by heating by use of heaters, and a resistance value of a heater for the first atomizing unit is larger than a resistance value of a heater for the second atomizing unit.

Another mode of the present invention comprises the flavor inhaler of the above mode, wherein the control unit stops electric conduction to the first atomizing unit when continuous electric conduction time of electric conduction to the first atomizing unit exceeds a first cut-off time, and stops electric conduction to the second atomizing unit when continuous electric conduction time of electric conduction to the second atomizing unit exceeds a second cut-off time, wherein the quantity of aerosol that is generated when the first atomizing unit is energized for a period of the first cut-off time is different from the quantity of aerosol that is generated when the second atomizing unit is energized for a period of the second cut-off time.

Another mode of the present invention comprises the flavor inhaler of the above mode, wherein the length of the first cut-off time is different from the length of the second cut-off time.

Another mode of the present invention comprises the flavor inhaler of the above mode, wherein the length of the first cut-off time is shorter than the length of the second cut-off time.

Another mode of the present invention comprises the flavor inhaler of the above mode, wherein the control unit changes the quantity of aerosol to be generated in at least one of the first atomizing unit and the second atomizing unit, by changing one of: electric power to be supplied to the respective atomizing units, and electric conduction time for the respective atomizing units.

Another mode of the present invention comprises the flavor inhaler of the above mode, wherein the control unit changes the quantity of aerosol to be generated in at least one of the first atomizing unit and the second atomizing unit, by changing both of: electric power to be supplied to the respective atomizing units, and electric conduction time for the respective atomizing units.

Another mode of the present invention comprises the flavor inhaler of the above mode, wherein the control unit determines the quantity of aerosol to be generated in at least one of the first atomizing unit and the second atomizing unit; determines, based on the determined quantity of aerosol to be generated, the electric energy to be supplied to the corresponding atomizing unit; selects, from plural combinations of a voltage to be applied and electric conduction time for the atomizing unit for obtaining the electric energy, a combination which is covered by a predetermined variable range of electric conduction time or a predetermined variable range of a voltage to be applied; and drives the corresponding atomizing unit with the selected voltage to be applied and the selected electric conduction time.

Another mode of the present invention comprises the flavor inhaler of the above mode, wherein the control unit determines the quantity of aerosol to be generated in at least one of the first atomizing unit and the second atomizing unit; determines, based on the determined quantity of aerosol to be generated, the electric energy to be supplied to the corresponding atomizing unit; determines, based on a predetermined fixed electric conduction time, a voltage to be applied to the corresponding atomizing unit for obtaining the electric energy; and drives the corresponding atomizing unit with the determined voltage to be applied and the fixed electric conduction time.

Another mode of the present invention comprises the flavor inhaler of the above mode, wherein the control unit determines a combination of a voltage to be applied and electric conduction time for at least one of the first atomizing unit and the second atomizing unit; calculates, based on the combination, electric energy to be supplied to the corresponding atomizing unit; and, in the case that a calculated value of the electric energy exceeds a predetermined upper limit value, drives the corresponding atomizing unit with a voltage to be applied and electric conduction time that satisfy electric energy of the predetermined upper limit value.

Another mode of the present invention comprises the flavor inhaler of the above mode, wherein the upper limit value decreases as the voltage to be applied increases.

Another mode of the present invention comprises the flavor inhaler of the above mode, wherein the control unit can change the quantity of aerosol to be generated in the first atomizing unit by changing the time of electric conduction to the first atomizing unit, and the quantity of aerosol to be generated in the second atomizing unit by changing the electric power to be supplied to the second atomizing unit.

Another mode of the present invention comprises the flavor inhaler of the above mode, wherein the control unit can change the quantity of aerosol to be generated in the second atomizing unit; and an upper limit of a variable range of the quantity of aerosol to be generated in the second atomizing unit is larger than the quantity of aerosol to be generated in the first atomizing unit, and a lower limit of the variable range of the quantity of aerosol to be generated in the second atomizing unit is smaller than the quantity of aerosol to be generated in the first atomizing unit.

Another mode of the present invention comprises the flavor inhaler of the above mode, wherein the control unit can change the quantity of aerosol to be generated in the first atomizing unit, and a lower limit of the variable range of the quantity of aerosol to be generated in the first atomizing unit is larger than zero.

Another mode of the present invention comprises the flavor inhaler of the above mode, wherein the variable range of the quantity of aerosol to be generated in the first atomizing unit includes zero.

Another mode of the present invention comprises the flavor inhaler of the above mode, wherein the control unit can change both the quantity of aerosol to be generated in the first atomizing unit and the quantity of aerosol to be generated in the second atomizing unit, and the width of the variable range of the quantity of aerosol to be generated in the first atomizing unit is narrower than the width of the variable range of the quantity of aerosol to be generated in the second atomizing unit.

Another mode of the present invention comprises the flavor inhaler of the above mode, wherein control unit can change both the quantity of aerosol to be generated in the first atomizing unit and the quantity of aerosol to be generated in the second atomizing unit, and the variable range of the quantity of aerosol to be generated in the first atomizing unit is included in a range between a lower limit value and an upper limit value of the variable range of the quantity of aerosol to be generated in the second atomizing unit.

Another mode of the present invention comprises the flavor inhaler of the above mode, wherein the control unit determines, based on an accumulated quantity of aerosol passed through the first flow path, the electric energy to be supplied to the first atomizing unit for increasing the quantity of aerosol to be generated in the first atomizing unit.

Another mode of the present invention comprises the flavor inhaler of the above mode, wherein the control unit determines, based on an accumulated quantity of aerosol passed through the first flow path, the electric energy to be supplied to the second atomizing unit for decreasing the quantity of aerosol to be generated in the second atomizing unit.

Another mode of the present invention comprises the flavor inhaler of the above mode, wherein the control unit obtains, based on an accumulated quantity of electric energy supplied to the first atomizing unit, the accumulated quantity of aerosol.

Another mode of the present invention comprises the flavor inhaler of the above mode, and further comprises a mixing chamber for mixing the aerosol flown through the first flow path with the aerosol flown through the second flow path, and the mixing chamber is communicated with the mouthpiece.

Another mode of the present invention comprises the flavor inhaler of the above mode, wherein the mixing chamber has a cross-sectional area larger than any of the cross-sectional areas of the first flow path and the second flow path.

Another mode of the present invention comprises the flavor inhaler of the above mode, wherein at least one of the first flow path and the second flow path comprises plural flow paths.

Another mode of the present invention comprises the flavor inhaler of the above mode, wherein the first flow path and the second flow path are arrange in parallel to each other.

ADVANTAGEOUS EFFECTS OF INVENTION

According to the present invention, the quantity of aerosol and the quantity of favor to be inhaled are controlled independently from each other.

BRIEF DESCRIPTION OF DRAWINGS

Fig. 1 is a configuration diagram of a flavor inhaler 100 according to an embodiment.
Fig. 2 is a flow chart showing operation of a control unit 130 relating to control in a first mode.
Fig. 3A is a flow chart showing an example of operation of a control unit 130 for driving atomizing units 104 for obtaining desired quantities of generated aerosol.
Fig. 3B is a flow chart showing an example of control for driving, based on electric conduction time t and an applied voltage V, a heater for each atomizing unit 104.
Fig. 4A is a figure for explaining a method for determining electric conduction time t and an applied voltage V for a heater for an atomizing unit 104, in the case that a variable range $T_{Range}$ of electric conduction time has been set.
Fig. 4B is a figure for explaining a method for determining electric conduction time t and an applied voltage V for the heater for the atomizing unit 104, in the case that a variable range $V_{Range}$ of applied voltages has been set.
Fig. 5 is a flow chart showing operation of a control unit 130 relating to control in a second mode.
Fig. 6A is a figure for explaining the electric energy supplied to a heater for an atomizing unit 104, in the case that electric conduction time t is designated by a user via a user setting unit 150.
Fig. 6B is a figure for explaining the electric energy supplied to the heater for the atomizing unit 104, in the case that an applied voltage V is designated by a user via the user setting unit 150.
Fig. 7 is a figure for explaining a modified example of control performed in the case that an applied voltage V is designated by a user via the user setting unit 150.
Fig. 8 is a flow chart showing operation of a control unit 130 relating to control in a third mode.

Fig. 9 is a flow chart showing operation of a control unit 130 relating to control in a fourth mode.

Fig. 10 is a block diagram showing a construction of an external device 200 which can be used together with the flavor inhaler 100.

Fig. 11 shows an example of a user interface screen 245 displayed on a display 240 in the external device 200.

## DESCRIPTION OF EMBODIMENTS

In the following description, embodiments of the present invention will be explained with reference to the figures.

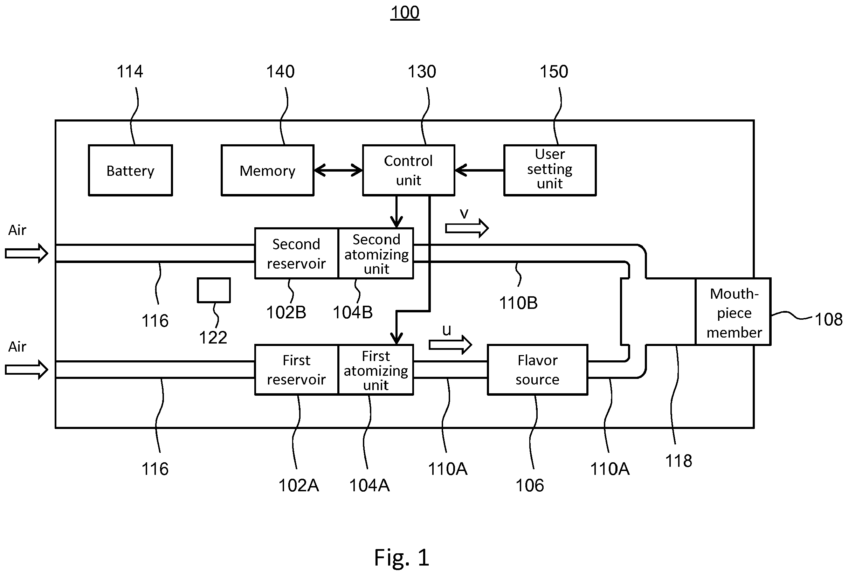

Fig. 1 is a configuration diagram of a flavor inhaler 100 according to an embodiment. It should be reminded that Fig. 1 shows respective elements included in the flavor inhaler 100 in a schematic and conceptual manner, and does not show precise arrangement, shapes, sizes, positional relationship, and so on of the respective elements and the flavor inhaler 100.

As shown in Fig. 1, the flavor inhaler 100 comprises reservoirs 102 (a first reservoir 102A and a second reservoir 102B), atomizing units 104 (a first atomizing unit 104A and a second atomizing unit 104B), a flavor source 106, a mouthpiece member 108, aerosol flow paths 110 (a first aerosol flow path 110A and a second aerosol flow path 110B), and a mixing chamber 118. Some of these elements in the flavor inhaler 100 may be gathered together to form a cartridge which is constructed to be attachable/detachable. For example, the flavor source 106 only may be constructed as a cartridge which is attachable/detachable to/from a main body of the flavor inhaler 100; the atomizing units 104 and the reservoirs 102 may be constructed as a cartridge which is attachable/detachable to/from a battery 114; and the flavor source 106, the reservoirs 102, and the atomizing units 104 may be integrated into a cartridge which is attachable/detachable to/from a battery 114.

The reservoirs 102 (a first reservoir 102A and a second reservoir 102B) hold aerosol sources. For example, the reservoirs 102 comprise fibrous or porous material, and hold the aerosol sources, each of which is in the form of fluid, by spaces between fibers or in pores in the porous material. Alternatively, the reservoirs 102 may be constructed as tanks for storing fluid in states that the fluid can be flown. The aerosol source may be, for example, a liquid such as glycerin or propylene glycol. The reservoirs 102 comprise constructions for allowing replenishment of the aerosol sources, or constructions for allowing replacement of the reservoirs themselves when the aerosol sources are exhausted.

The atomizing units 104 (the first atomizing unit 104A and the second atomizing unit 104B) are constructed to generate aerosol by atomizing the aerosol sources. Each atomizing unit 104 generates the aerosol when inhaling action of a user is detected by a puff sensor 122 (for example, a pressure sensor for detecting change in pressure in an air intake channel 116 or the aerosol flow path 110, or a manipulation button which can be manipulated by a user). For example, a wick, which is not shown, is arranged for connection between the first reservoir 102A and the first atomizing unit 104A. A part of the wick extends to the inside of the first reservoir 102A and is in contact with the aerosol source. Another part of the wick extends toward the first atomizing unit 104A. The aerosol source is sent from the first reservoir 102A to the first atomizing unit 104A by capillary effect in the wick. Similarly, another wick, which is not shown, is arranged for connection between the second reservoir 102B and the second atomizing unit 104B. Each atomizing unit 104 comprises, for example, a heater which is electrically connected to the battery 114. The heater for each atomizing unit 104 is arranged to be in contact with the wick for the atomizing unit 104, and the aerosol source sent through the wick is heated to be atomized. Another example of each atomizing unit 104 may be an ultrasonic-type atomizer which atomizes the aerosol source by ultrasonic vibration. The air intake channel 116 is connected to each atomizing unit 104, and the air intake channel 116 leads to the outside of the flavor inhaler 100. The aerosol generated in the first atomizing unit 104A and the aerosol generated in the second atomizing unit 104B are mixed with air, which is taken via the air intake channel 116, and sent to the first aerosol flow path 110A and the second aerosol flow path 110B, respectively.

The flavor source 106 is a unit for providing aerosol with flavor. The flavor source 106 is arranged in a middle position in the first aerosol flow path 110A. A fluid comprising a mixture of air and the aerosol generated in the first atomizing unit 104A (in the following description, it should be reminded that this fluid mixture may simply be referred to as aerosol) flows through the first aerosol flow path 110A to the side of a mouthpiece (the mouthpiece member 108) as a result of inhaling action by a user. That is, in the point of view of the flow of the aerosol, the flavor source 106 is arranged in a position downstream the first atomizing unit 104A. In other words, in the aerosol flow path 110, the position of the flavor source 106 is closer to the mouthpiece than the position of the first atomizing unit 104A. In this manner, the aerosol generated in the first atomizing unit 104A passes through the flavor unit 106 and arrives at the mouthpiece. When the aerosol passes through the flavor source 106, flavor components from the flavor source 106 are added to the aerosol. For example, the flavor source 106 may be that which originates from tobacco, such as shredded tobacco, a product which is made by processing raw material comprising tobacco to have a granular form, a sheet form, or a powder form, or the like, or that which does not originate from tobacco, such as a product made by use of a plant other than tobacco (for example, mint, a herb, and so on). For example, the flavor source 106 comprises a nicotine component. The flavor source 106 may comprise a flavor component such as menthol. Note that, in addition to having the flavor source 106, it is possible to make the reservoir 102 (one or both of the first reservoir 102A and the second reservoir 102B) to have a material comprising a flavor component. For example, the flavor inhaler 100 may be constructed in such a manner that the flavor source 106 holds flavor material which originates from tobacco and each reservoir 102 comprises flavor material which does not originate from tobacco.

Each aerosol flow path 110 is a tubular structure for sending the fluid mixture comprising air and the aerosol generated in each atomizing unit 104 to the mixing chamber 118. As shown in Fig. 1, the aerosol flow paths 110 comprise a first aerosol flow path 110A and a second aerosol flow path 110B. The first aerosol flow path 110A connects between the first atomizing unit 104A and the mixing chamber 118, and the second aerosol flow path 110B connects between the second atomizing unit 104B and the mixing chamber 118. As shown in the figure, the first aerosol flow path 110A and the second aerosol flow path 110B are arranged in parallel with each other. At least one of the first aerosol flow path 110A and the second aerosol flow path 110B may be constructed to include plural flow paths. As explained above, the flavor source 106 is arranged in a position in the middle of the first aerosol flow path 110A. That is, the first aerosol flow path 110A connects between the first atomizing unit 104A and the mixing chamber 118 via the flavor source 106. Accordingly, the aerosol generated in the first atomizing unit 104A is sent together with air to the first aerosol flow path 110A, flavor components are added thereto when passing through the flavor source 106, and it is sent to the mixing chamber 118. On the other hand, the second aerosol flow path 110B connects between the second atomizing unit 104B and the mixing chamber 118 in a direct manner, i.e., without passing through the flavor source 106. Accordingly, the aerosol generated in the second atomizing unit 104B and sent to the second aerosol flow path 110B does not pass through the flavor source 106; thus, the aerosol is sent to the mixing chamber 118 without addition of the flavor components included in the flavor source 106. Note that a flavor source different from the flavor source 106 (for example, a flavor source which can add flavor components, which are different from the components which can be added by the flavor source 106, to the aerosol) may further be added to the second aerosol flow path 110B.

The mixing chamber 118 is positioned at ends (downstream-side ends) of the first aerosol flow path 110A and the second aerosol flow path 110B, and used for mixing the aerosol passed through the first aerosol flow path 110A and the aerosol passed through the second aerosol flow path 110B. For facilitating mixing of the aerosol, the mixing chamber 118 is constructed to have a flow-path cross-sectional area larger than the cross-sectional area of the first aerosol flow path 110A and the cross-sectional area of the second aerosol flow path 110B. Note that the cross-sectional area of the flow path is an area of a cross section, which is vertical to a direction of flow of aerosol (the directions of arrows u and v shown in Fig. 1), of an aerosol flow path.

The mouthpiece member 108 is a member which is connected to a downstream side of the mixing chamber 118, and constructed to make the aerosol in the mixing chamber 118 to be released toward the outside of the flavor inhaler 100. A user takes air including the aerosol into the mouth by holding the mouthpiece member 108 in the user's mouth and inhaling it. In this manner, the aerosol from the first aerosol flow path 110A and the aerosol from the second aerosol flow path 110B are flown into each other at the mixing chamber 118, and inhaled by a user from the mouthpiece member 108.

The flavor inhaler 100 according to this embodiment further comprises a control unit 130, a memory 140, and a user setting unit 150. The control unit 130 is an electronic circuit module constructed as a microprocessor or a microcomputer, and is programmed to control operation of the flavor inhaler 100 according to computer-executable instructions stored in the memory 140. The memory comprises an information storing medium such as a ROM, a RAM, a flash memory, or the like. The memory 140 stores, in addition to the computer-executable instructions, setting data which are necessary for controlling the flavor inhaler 100.

The user setting unit 150 allows a user to set the quantity of aerosol to be generated in each atomizing unit 104 (hereinafter, an aerosol generation quantity). For example, the user setting unit 150 is constructed as a button, a switch, a control, or the like which can be physically manipulated by a user. In another example, the user setting unit 150 may be constructed as a communication interface (for example, a USB terminal or a wireless interface) which receives an instruction from a user via communication connection with an external computer.

A user may be able to set the aerosol generation quantity u of the first atomizing unit 104A and the aerosol generation quantity v of the second atomizing unit 104B in an independent manner via the user setting unit 150, for example. In the case that the aerosol generation quantity u of the first atomizing unit 104A is set via the user setting unit 150 by manipulation thereof by the user, the control unit 130 controls the first atomizing unit 104A in such a manner that the first atomizing unit 104A performs operation in accordance with the setting. Similarly, in the case that the aerosol generation quantity v of the second atomizing unit 104B is set via the user setting unit 150 by manipulation thereof by the user, the control unit 130 controls the second atomizing unit 104B in such a manner that the second atomizing unit 104B performs operation in accordance with the setting. The setting made by use of the user setting unit 150 may be that for one of the aerosol generation quantity u of the first atomizing unit 104A and the aerosol generation quantity v of the second atomizing unit 104B, or that for changing the both quantities at the same time.

In this manner, a user can arbitrarily change the aerosol generation quantity of the first atomizing unit 104A and the aerosol generation quantity of the second atomizing unit 104B. The aerosol generation quantity of the first atomizing unit 104A and the aerosol generation quantity of the second atomizing unit 104B are changeable within their respective predetermined ranges (variable ranges). For example, the aerosol generation quantity of the second atomizing unit 104B may be changeable and the aerosol generation quantity of the first atomizing unit 104A may be fixed; and, regarding the variable range of the aerosol generation quantity of the second atomizing unit 104B, the upper limit thereof may be set to a value larger than the aerosol generation quantity (a fixed value) of the first atomizing unit 104A, and the lower limit thereof may be set to a value smaller than the aerosol generation quantity (the fixed value) of the first atomizing unit 104A. That is, compared with the aerosol generation quantity of the first atomizing unit 104A, the range within that the aerosol generation quantity of the second atomizing unit 104B can be changed is wider. Thus, the total quantity of aerosol to be supplied to a user can be changed within a wide range. Further, it may be constructed that the aerosol generation quantity of the first atomizing unit 104A is changeable; and, regarding the variable range of the aerosol generation quantity of the first atomizing unit 104A, the lower limit thereof may be set to a value larger than zero. That is, a predetermined quantity of aerosol, that is not zero, is always generated in the first atomizing unit 104A, and, accordingly, the generated aerosol is sent to the flavor source 106 and a predetermined non-zero quantity of flavor components is always supplied to a user. In another example, the aerosol generation quantity of the first atomizing unit 104A may be zero. For example, in the case that the second reservoir 102B has material including flavor components as explained above, it becomes possible to supply, to a user, flavor components included in the second reservoir 102B only, by setting the aerosol generation quantity of the first atomizing unit 104A to zero. In another example, it may be constructed that both the aerosol generation quantity of the first atomizing unit 104A and the aerosol generation quantity of the second atomizing unit 104B are changeable; and the width of the variable range of the aerosol generation quantity of the first atomizing unit 104A may be set to be narrower than the width of the variable range of the aerosol generation quantity of the second atomizing unit 104B, or the variable range of the aerosol generation quantity of the first atomizing unit 104A may be set to be that included in the range between the upper limit and the lower limit of the variable range of the aerosol generation quantity of the second atomizing unit 104B. Thereby, the total quantity of aerosol to be supplied to a user can be varied within a wide range, without largely changing the quantity of flavor components supplied to the user.

Instead of independently setting the aerosol generation quantity u of the first atomizing unit 104A and the aerosol generation quantity v of the second atomizing unit 104B as explained above, a user may be able to set, via the user setting unit 150, one or both of the total quantity p of aerosol to be inhaled (i.e., to be delivered to the mouthpiece member 108) and the quantity q of flavor components supplied from the flavor source 106 and included in the aerosol to be inhaled (hereinafter, it is simply referred to as the flavor component quantity). The total aerosol quantity p delivered to the mouthpiece member 108 is equal to a sum of the quantities of aerosol generated in both the first atomizing unit 104A and the second atomizing unit 104B, i.e., u+v. Further, as explained above, the flavor components supplied from the flavor source 106 and included in the aerosol delivered to the mouthpiece member 108 are those obtained as a result that the aerosol form the first atomizing unit 104A is made to be flown through the flavor source 106, thus, the quantity thereof (the flavor component quantity q) depends only on the aerosol generation quantity u of the first atomizing unit 104A. That is, the flavor component quantity q is a function of the aerosol generation quantity u of the first atomizing unit 104A, and can be represented as q=f(u) (provided that f(0)=0) (note that the function f may be that which takes effect of gradual deterioration of ability to supply flavor components from the flavor source 106, due to flow of aerosol passing through it, into consideration). Thus, if a combination of the flavor component quantity q and the total aerosol quantity p delivered to the mouthpiece member 108 is given, a combination of the aerosol generation quantity u of the first atomizing unit 104A and the aerosol generation quantity v of the second atomizing unit 104B is uniquely determined as shown by use of the formulas (1) and (2).

$$(1) \qquad u=f^{-1}(q)$$

$$(2) \qquad V=p-f^{-1}(q)$$

Accordingly, for example, in the case that both the total aerosol quantity p and the flavor component quantity q to the mouthpiece member 108 are set via the user setting unit 150 by manipulation thereof by the user, the control unit 130 determines, in accordance with the formulas (1) and (2) and by use of the set values, the aerosol generation quantities u and v of the first atomizing unit 104A and the second atomizing unit 104B, respectively, and controls the first atomizing unit 104A and the second atomizing unit 104B in such a manner that the first atomizing unit 104A and the second atomizing unit 104B perform operation in accordance with the determined values, respectively. Alternatively, for example, in the case that setting for clanging the flavor component quantity q only is performed via the user setting unit 150 by manipulation thereof by the user, the control unit 130 controls the first atomizing unit 104A in accordance with the formula (1) in such a manner that the aerosol generation quantity u of the first atomizing unit 104A is changed by a quantity corresponding to the change, and controls the second atomizing unit 104B in accordance with the formula (2) in such a manner that the aerosol generation quantity v of the second atomizing unit 104B is changed based on the change in the aerosol generation quantity u of the first atomizing unit 104A (for cancelling out the change in the aerosol generation quantity u by use of the change in the aerosol generation quantity v). Further, in another example, in the case that setting for clanging the total aerosol quantity p only is performed via the user setting unit 150 by manipulation thereof by the user, the control unit 130 controls the second atomizing unit 104B in accordance with the formula (2) in such a manner that the aerosol generation quantity v of the second atomizing unit 104B is changed by a quantity corresponding to the change. Regarding details of the above control, refer to the control in a first mode which will be explained later.

Further, in another example, instead of setting the aerosol generation quantity u of the first atomizing unit 104A and the aerosol generation quantity v of the second atomizing unit 104B, and setting the total aerosol quantity p and the flavor component quantity q to be inhaled as explained above, a user may directly set, via the user setting unit 150, one or both of the voltage V applied to the heater for the atomizing unit 104 and the time of electric conduction thereto. In the case that both the applied voltage V and the electric conduction time t are set, the electric energy W $(=(V^2/R)^*t)$ supplied to the heater for the atomizing unit 104 may be set directly, rather than setting the applied voltage V and the electric conduction time t independently. Note that R is a resistance value of the heater. In the case that either one of the voltage V applied to the heater for the atomizing unit 104 (the first atomizing unit 104A or the second atomizing unit 104B) and the time of electric conduction thereto is set via the user setting unit 150 by manipulation thereof by the user, the control unit 130 controls the atomizing unit 104 in accordance with the setting. Regarding details of the control in the above example, refer to the control in a second mode which will be explained later.

As explained above, a user can perform setting with respect to the user setting unit 150 by use of some different methods, and, in any of the cases, the control unit 130 controls the aerosol generation quantity u of the first atomizing unit 104A and the aerosol generation quantity v of the second atomizing unit 104B in an independent manner, in response to the setting made by the user. In the following description, examples of control performed by the control unit 130 will be explained in detail.

< Control in a first mode >

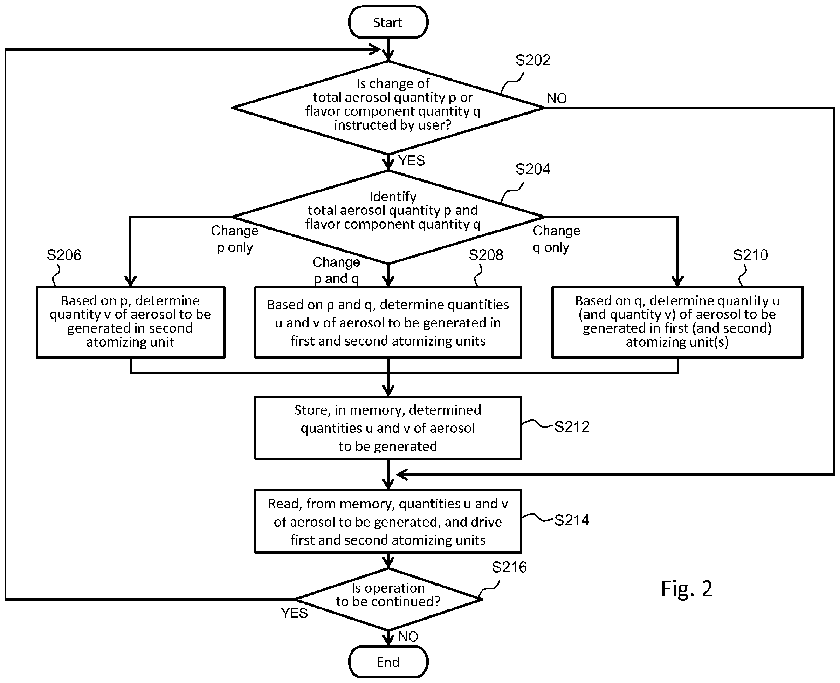

Fig. 2 is a flow chart showing operation of a control unit 130 relating to control in a first mode. The control in the first mode is an example of control in the case that one or both of the total aerosol quantity p and the flavor component quantity q is/are set via a user setting unit 150.

First, in step S202, the control unit 130 judges whether or not a user instruction for changing at least one of the total aerosol quantity p and the flavor component quantity q has been inputted via the user setting unit 150. The total aerosol quantity p and the flavor component quantity q represent target values of the total aerosol quantity and the flavor component quantity generated in the flavor inhaler 100.

In the case that no user instruction for changing at least one of the total aerosol quantity p and the flavor component quantity q has been inputted via the user setting unit 150, the process proceeds to step S214. In step S214, the control unit 130 reads, from a memory 140, respective set values of the aerosol generation quantity u of the first atomizing unit 104A and the aerosol generation quantity v of the second atomizing unit 104B, and drives the first atomizing unit 104A and the second atomizing unit 104B in accordance with the read set values, respectively. For example, initial set values of respective quantities of aerosol to be generated in the first atomizing unit 104A and the second atomizing unit 104B, which are used when starting operation of the flavor inhaler 100, are stored in the memory 140 in advance. After starting operation of the flavor inhaler 100 and until a user instruction is inputted for the first time, the control unit 130 reads, from the memory 140, the initial set values of the respective quantities of aerosol to be generated, and drives the first atomizing unit 104A and the second atomizing unit 104B. As a result thereof, the flavor inhaler 100 performs, as a single atomizing action corresponding to a single inhaling action of a user, operation for supplying, to a user, a fixed (constant) flavor component quantity q and a fixed (constant) total aerosol quantity p, that are related by use of relationship between the initial set values of the respective quantities of aerosol to be generated and the formulas (1) and (2). Thereafter, in step S216, the control unit 130 determines whether operation of the flavor inhaler 100 should be continued (whether a next single atomizing action corresponding to a next single inhaling action of the user should be performed); and, in the case that the operation is to be continued, the process returns to step S202. For example, in the case that a detected value obtained from a puff sensor (a pressure sensor) 122 is lower than a predetermined threshold value, it can be judged that the user is attempting to perform a next inhaling action, so that the process starting from step S202 is repeated. Note that step S216 is optional, so that it may be omitted.

On the other hand, in step S202, in the case that it is judged that a user instruction for changing at least one of the total aerosol quantity p and the flavor component quantity q has been inputted via the user setting unit 150, the process proceeds to step S204, and the control unit 130 judges whether the user instruction inputted via the user setting unit 150 is an instruction for changing the total aerosol quantity p only, an instruction for changing the flavor component quantity q only, or an instruction for changing both the total aerosol quantity p and the flavor component quantity q. In the case that the user instruction is an instruction for changing the total aerosol quantity p only, the process proceeds to step S206; in the case that the user instruction is an instruction for changing both the total aerosol quantity p and the flavor component quantity q, the process proceeds to step S208; and, in the case that the user instruction is an instruction for changing the flavor component quantity q only, the process proceeds to step S210. The judgment in step S204 may be performed at any timing, for example, 1) when an input of a user instruction is detected by the control unit 130, 2) after completion of a single atomizing action, 3) during a predetermined time lag period from a point in time when an inhaling action is detected by the puff sensor 122 and until a point in time when atomizing of aerosol is started, or 4) during atomizing action (during a period of electric conduction to the heater).

If it is judged, as a result of judgment in step S204, that the user instruction is an instruction for changing the total aerosol quantity p only, the control unit 130 determines, in step S206, the aerosol quantity v to be generated, after the change, in the second atomizing unit 104B, in accordance with the total aerosol quantity p relating to the user instruction, and based on the formula (2). Next, in step S212, the control unit 130 updates the set value of the aerosol generation quantity of the second atomizing unit 104B, that is stored in the memory 140, by use of the value of the new aerosol generation quantity v determined in step S206. Further, in step S214, the control unit 130 reads respective set values of the aerosol generation quantity u of the first atomizing unit 104A and the aerosol generation quantity v of the second atomizing unit 104B out of the memory 140, and drives the first atomizing unit 104A and the second atomizing unit 104B in accordance with the read set values, respectively. More specifically, the control unit 130 drives the second atomizing unit 104B in accordance with the set value of the new aerosol generation quantity v determined in step S206; and drives the first atomizing unit 104A in accordance with the aerosol generation quantity u of the first atomizing unit 104A that is maintained to be the same value according to relationship with formula (1), since change with respect to the flavor component quantity q is not included in the user instruction. As a result thereof, the total aerosol quantity p to be delivered to the mouthpiece member 108 is changed in response to the user instruction to the user setting unit 150, and the flavor component quantity q delivered to the mouthpiece member 108 is maintained to be constant. Thus, the flavor inhaler 100 can vary the total aerosol quantity p while maintaining the flavor component quantity q supplied to a user to be constant. Thereafter, in step 206, the control unit 130 determines, as explained above, whether operation of the flavor inhaler 100 should be continued, and the process returns to step S202 in the case that the operation is to be continued.

If it is judged, as a result of judgment in step S204, that the user instruction is an instruction for changing both the total aerosol quantity p and the flavor component quantity q, the control unit 130 determines, in step S208, the aerosol generation quantity u of the first atomizing unit 104A and the aerosol generation quantity v of the second atomizing unit 104B, in accordance with the total aerosol quantity p and the flavor component quantity q relating to the user instruction, and based on the formulas (1) and (2). Next, in step S212, the control unit 130 updates the set values of the aerosol generation quantities of the first atomizing unit 104A and the second atomizing unit 104B, that are stored in the memory 140, by use of the values of the new aerosol generation quantities u and v determined in step S208, respectively. Further, in step S214, the control unit 130 reads respective set values of the aerosol generation quantity u of the first atomizing unit 104A and the aerosol generation quantity v of the second atomizing unit 104B out of the memory 140, and drives the first atomizing unit 104A and the second atomizing unit 104B in accordance with the read set values, respectively. As a result thereof, the total aerosol quantity p and the flavor component quantity q to be delivered to the mouthpiece member 108 are changed in response to the user instruction to the user setting unit 150. Thus, the flavor inhaler 100 can adjust, in an independent manner, the total aerosol quantity p and the flavor component quantity q supplied to a user. Thereafter, in step 206, the control unit 130 determines, as explained above, whether operation of the flavor inhaler 100 should be continued, and the process returns to step S202 in the case that the operation is to be continued.

If it is judged, as a result of judgment in step S204, that the user instruction is an instruction for changing the flavor component quantity q only, the control unit 130 determines, in step S210, the aerosol quantity u to be generated in the first atomizing unit 104A after the change, in accordance with the flavor component quantity q relating to the user instruction, and based on the formula (1). Next, in step S212, the control unit 130 updates the set value of the aerosol generation quantity of the first atomizing unit 104A, that is stored in the memory 140, by use of the value of the new aerosol generation quantity u determined in step S210. Further, in step S214, the control unit 130 reads respective set values of the aerosol generation quantity u of the first atomizing unit 104A and the aerosol generation quantity v of the second atomizing unit 104B out of the memory 140, and drives the first atomizing unit 104A and the second atomizing unit 104B in accordance with the read set values, respectively. Although the total aerosol quantity p is changed to correspond to the change in the aerosol generation quantity u in the case that the condition of operation of the first atomizing unit 104A only is changed in this manner, i.e., although an instruction for changing the total aerosol quantity p only is inputted to the user setting unit 150, there may be a case that a user senses an unnatural feel that an unexpected change in the total quantity of inhaled aerosol has occurred. For avoiding the above matter, the control unit 130 may further perform, as an option, control for changing the aerosol generation quantity v of the second atomizing unit 104B based on the formula (2), and updating the set value of the aerosol generation quantity of the second atomizing unit 104B, that is stored in the memory 140, by use of the new value obtained after the change. As a result thereof, the flavor component quantity q supplied to the user can be changed in response to the user instruction to the user setting unit 150, while maintaining the total aerosol component quantity p supplied to the user to be constant. Thereafter, in step 206, the control unit 130 determines, as explained above, whether operation of the flavor inhaler 100 should be continued, and the process returns to step S202 in the case that the operation is to be continued.

After the new aerosol generation quantity u and/or the new aerosol generation quantity v are determined in accordance with the user instruction given via the user setting unit 150 in step S206, step S208, or step S210 as explained, the set values/value of the quantities/quantity of aerosol generation in the memory 140 are/is updated by the values/value of the quantity u and/or the quantity v. Thus, in the case that the process has returned to step S202 thereafter (i.e., after one of step S206, step S208, and step S210 is completed) for continuing operation of the flavor inhaler 100, and proceeded to step S214 in response to the state that no user instruction for changing at least one of the total aerosol quantity p and the flavor component quantity q has been inputted via the user setting unit 150, the most recently updated set values/value of the aerosol generation quantities/quantity u and/or v are/is read out of the memory 140, and the first atomizing unit 104A and the second atomizing unit 104B are driven in accordance with the new set values, respectively. As a result thereof, the flavor inhaler 100 operates to supply the fixed (constant) total aerosol quantity p and the fixed (constant) flavor component quantity q, that are determined based on the last-inputted user instruction, to the user, until a next user instruction for changing at least one of the total aerosol quantity p and the flavor component quantity q is inputted via the user setting unit 150.

Note that, when new aerosol generation quantities/quantity u and/or v are/is determined in accordance with a user instruction via the user setting unit 150 in step S206, step S208, or step S210, there may be a case that the determined aerosol generation quantities u and/or v exceed the maximum aerosol quantities that can be generated by the respective atomizing units 104A and 104B (the maximum aerosol quantities that are determined, for example, based on the heating capacity of each heater, electric power that can be supplied from a battery, and so on). For dealing with the above matter, the control unit 130 may be constructed in such a manner that it notifies a user of an error, and prompts the user to repeat step S202 for inputting appropriate values of the total aerosol quantity p and the flavor component quantity q. Alternatively, the control unit 130 may be constructed in such a manner that it drives the heaters for the respective atomizing units 104A and 104B, by applying thereto the maximum electric energy $W_{max}$ that is similar to that in step S512, that will be explained later, for making the respective atomizing units 104A and 104B to generate the maximum quantities of aerosol. Further, there may be the case that the aerosol generation quantities/quantity u and/or v, that are/is determined in accordance with a user instruction via the user setting unit 150, are/is zeros/zero. In such a case, the control unit 130 performs control for stopping generation of aerosol in the atomizing units 104 (one or both of the first atomizing unit 104A and the second atomizing unit 104B) which have been instructed to generate the aerosol quantities of zeros. As a result thereof, the quantity of aerosol to be generated in the subject atomizing unit 104 is made to be zero.

When driving the first atomizing unit 104A or the second atomizing unit 104B in step S214, the control unit 130 controls the quantity of aerosol to be generated in each atomizing unit 104 to have a desired value, by changing the electric energy W supplied from the battery 114 to each atomizing unit 104. This is based on the fact that the quantity of aerosol to be generated is usually determined based on energy applied to the aerosol source. For example, data representing relationship between the electric energy supplied to a heater for each atomizing unit 104 and the quantities of aerosol generated from the aerosol source when the heater is heated by the electric energy (hereinafter, this is referred to as aerosol generation quantity characteristic data) is stored in the memory 140 in advance. The control unit 130 obtains a value of the electric energy to be supplied to the heater, which corresponds to each of set values of the aerosol generation quantities u and v, by referring to the aerosol generation quantity characteristic data, and controls the electric energy W to be supplied from the battery 114 to the heater for each atomizing unit 104 in such a manner that the value of the electric energy W coincides with the obtained value.

For controlling the electric energy W (= (electric power P) * (electric conduction time t)) supplied to the heater for the atomizing units 104, the control unit 130 may change either one of the supplied electric power P and the electric conduction time t (another of them is fixed), or may change both the supplied electric power P and the electric conduction time t at the same time. Alternatively, in both the first atomizing unit 104A and the second atomizing unit 104B, the electric energy W may be changed by changing electric power P to be supplied to the heaters; or, in both the first atomizing unit 104A and the second atomizing unit 104B, the electric energy W may be changed by changing electric conduction time t relating to the heaters. Further, it may be possible to change, in the first atomizing unit 104A, the electric energy W by changing one of the supplied electric power P and the electric conduction time t relating to the heater, and change, in the second atomizing unit 104B, the electric energy W by changing another of the above.

In a preferable example, in the first atomizing unit 104A, the electric energy W is changed by changing the electric conduction time t relating to the heater, and, in the second atomizing unit 104B, the electric energy W is changed by changing the electric power P supplied to the heater (actually, the voltage V applied to the heater). In the above case, if it is supposed that the supplied electric power P and the electric conduction time t have similar degrees of control errors (deviation from target values) when driving a heater, there is relationship that $W = P \cdot t = (V^2/R) \cdot t$ (R is a resistance value of the heater) and, thus, W is proportional to $V^2$ and proportional to t; so that change in the electric energy supplied to the heater for the first atomizing unit 104A (i.e., change in the quantity of aerosol generated in the first atomizing unit 104A) is smaller than change in the electric energy supplied to the heater for the second atomizing unit 104B (i.e., change in the quantity of aerosol generated in the second atomizing unit 104B). Thus, change in the flavor component quantity q supplied to a user (as explained above, this depends only on the aerosol generation quantity u of the first atomizing unit 104A), that is due to a control error, can be made to be smaller than change in the total aerosol quantity p.

Note that, for the reason similar to that explained above, it is preferable to select, for the resistance value $R_1$ of the heater for the first atomizing unit 104A, a value larger than that of the resistance value $R_2$ of the heater for the second atomizing unit 104B. In the above case, since W is inversely proportional to R since there is the relationship $W = (V^2/R) \cdot t$, the range of change in electric energy supplied to the heater for the second atomizing unit 104B becomes larger than the range of change in electric energy supplied to the heater for the first atomizing unit 104A, in a manner similar to the above manner, if the variable range of the applied voltage V and that of the electric conduction time t are similar. Thus, compared with the change in the flavor component quantity q supplied to a user, the change in the total aerosol quantity p can easily be made to be larger.

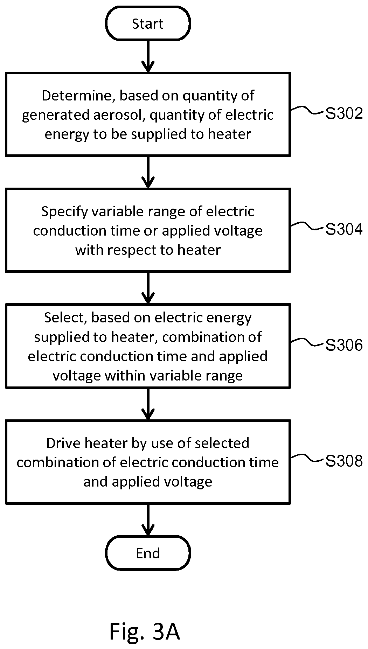

Fig. 3A is a flow chart showing an example of operation of the control unit 130 for driving the atomizing units 104 for obtaining desired aerosol generation quantities. The control based on this flow chart is that showing, in a more tangible manner, the process in step S214 in the flow chart in Fig. 2, and is applicable to the control for the first atomizing unit 104A and the control for the second atomizing unit 104B without discrimination.

First, in step S302, the control unit 130 determines electric energy W to be supplied to the heaters for the atomizing units 140, based on set values of the aerosol generation quantities read out of the memory 140. For example, the control unit 130 obtains (i.e., determines) the values of electric energy supplied to the heaters, that correspond to the set values of the aerosol generation quantities, by referring to the aerosol generation quantity characteristic data stored in advance in the memory 140.

Next, in step S304, the control unit 130 specifies the variable range $t_{Range}$ of the electric conduction time t or the variable range $V_{Range}$ of the applied voltage V relating to the heater for each atomizing unit 104. For example, data representing the variable range $t_{Range}$ of the electric conduction time and the variable range $V_{Range}$ of the applied voltage are stored in the memory 140 in advance, and the data representing the variable range $t_{Range}$ or $V_{Range}$ is read out of the memory 140 by the control unit 130. For example, the variable range $t_{Range}$ of the electric conduction time is a predetermined range of a time period (for example, a range of 1.0-2.5 seconds) that is equal to or less than the time (for example, 3.0 seconds) that is generally considered as time required for a single inhaling action of a user, and the range shows that changing of time t of electric conduction to the heater is allowed within the range. In another example, the width of $t_{Range}$ may be zero, and time t of electric conduction to the heater may be fixed to a predetermined single value (for example, 2.0 seconds). This fixed electric conduction time may be updated, by studying a profile of inhaling by a user (inhaling time, etc.), for example. Similarly, the variable range $V_{Range}$ of the applied voltage shows that the voltage V applied to the heater can be changed within the range. The variable range $V_{Range}$ may be set appropriately based on the type of the battery 114 and so on; and, in the case that a lithium ion battery, for example, is used as the battery 114, the range may be set to the range of 3.2 V-4.2 V, for example, by using voltage control performed by use of a DC-DC converter or the like. Further, the variable range $V_{Range}$ may be realized by using pulse width modulation (PWM) control in such a manner that the duty ratio of the voltage V applied to the heater is changed, for example, in the range of 20 %-100 %. Note that step S304 may be performed before step S302.

Note that the reason for setting the variable range $t_{Range}$ of the electric conduction time t is as follows. In the case that the time t of electric conduction to the heater is lower than the lower limit of the variable range $t_{Range}$, electric conduction to the heater may be stopped before a desired quantity of supplied electric energy (that is determined in step S302) is obtained, even if the maximum voltage is applied to the heater, and, as a result, air only is delivered to a user during most of the period of the inhaling time, so that there may be a risk that the user senses an unnatural feel; on the other hand, in the case that the time t of electric conduction to the heater is higher than the lower limit of the variable range $t_{Range}$, such a risk can be eliminated, and the desired quantity of supplied electric energy can be surely obtained by selecting an appropriate value of the applied voltage. Further, in the case that the time t of electric conduction to the heater is higher than the upper limit of the variable range $t_{Range}$, there may be a risk that the length of time to drive the heater exceeds the length of time that is generally considered as that required for a single inhaling action of a user; thus, there may be a risk that the inhaling action of the user is completed before completing delivering of the total aerosol quantity or the flavor component quantity set by the user. On the other hand, in the case that the time t of electric conduction to the heater is lower than the upper limit of the variable range $t_{Range}$, it is possible to prevent a user from sensing an unnatural feel, and complete delivering of the set total aerosol quantity or the set flavor component quantity during the inhaling action of the user. Similarly, the reason for setting the variable range $V_{Range}$ of the applied voltage V is as follows. In the case that the voltage V applied to the heater is lower than the lower limit of variable range $V_{Range}$, the quantity of heat generated by the heater per unit time may be insufficient so that there may be a risk that generation of aerosol in an appropriate manner may be hindered; on the other hand, in the case that the voltage V applied to the heater is higher than the lower limit of variable range $V_{Range}$, it is possible to provide the heater with a sufficient quantity of heat per unit time, so that generation of aerosol can be performed in an appropriate manner. Further, in the case that the voltage V applied to the heater is higher than the upper limit of variable range $V_{Range}$, there may be a risk that the supplied electric energy exceeds the desired electric energy even if the time of electric conduction to the heater is set to be the shortest time; on the other hand, in the case that the voltage V applied to the heater is lower than the upper limit of variable range $V_{Range}$, the desired supplied electric energy can be surely obtained by selecting appropriate electric conduction time.

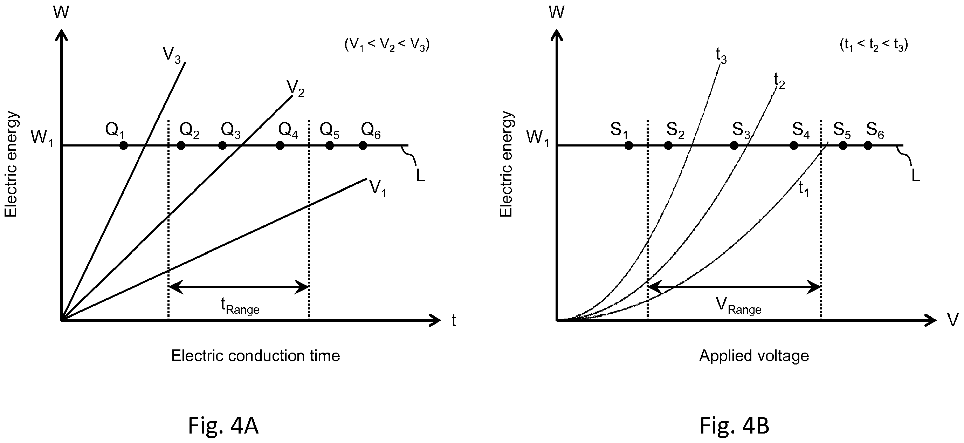

Next, in step S306, based on the electric energy to be supplied to the heater of the atomizing unit 104, that is determined in step S302, the control unit 130 selects, from combinations of electric conduction time t's and applied voltages V's that satisfy the relational formula of electric energy $W=(V^2/R)*t$, a combination of t and V wherein the electric conduction time t is that in the variable range $t_{Range}$ (Fig. 4A), or a combination of t and V wherein the applied voltage V is that in the variable range $V_{Range}$ (Fig. 4B).

Fig. 4A is a figure for explaining a method for determining electric conduction time t and an applied voltage V for the heater for the atomizing unit 104, in the case that the variable range $T_{Range}$ of electric conduction time has been set. As shown in Fig. 4A, when the applied voltage V is used as a parameter, the electric energy W supplied to the heater for the atomizing unit 104 is represented by a linear function of time t of electric conduction to the heater. The electric energy supplied to the heater (represented by $W_1$), that is determined in step S302, is drawn as a horizontal straight line L in Fig. 4A. If the value that can be used as the value of the electric conduction time is not limited to that within the variable range $t_{Range}$, a combination of the electric conduction time t and the applied voltage V corresponding to any point on the straight line L (for example, $Q_1$, $Q_2$, $Q_3$, $Q_4$,

$Q_5$, $Q_6$) can be used for obtaining the electric energy $W_1$. However, since the electric conduction time is limited to that within the variable range $t_{Range}$, the control unit 130 adopts (selects) only a combination of the electric conduction time t and the applied voltage V corresponding to a point on a line that is in the straight line L and delimited by the range $t_{Range}$ on the horizontal line (electric conduction time) (for example, $Q_2$, $Q_3$, $Q_4$), as set values usable for driving the atomizing unit 104 by the electric energy $W_1$. Note that, in the case that the width of the variable range $t_{Range}$ is zero and the value of the time t of electric conduction to the heater is fixed to a single value (for example, 2.0 seconds), the value of the voltage V applied to the heater, by which the electric energy $W_1$ can be obtained, can be uniquely determined from the fixed single value, as apparent from Fig. 4A (or as apparent from the relational formula $W=(V^2/R)*t$).

Similarly, Fig. 4B is a figure for explaining a method for determining electric conduction time t and an applied voltage V for the heater for the atomizing unit 104, in the case that the variable range $V_{Range}$ of applied voltages has been set. As shown in Fig. 4B, when the electric conduction time t is used as a parameter, the electric energy W supplied to the heater for the atomizing unit 104 is represented by a quadratic function of the voltage V applied to the heater. In a manner similar to that in the case of Fig. 4A, the control unit 130 adopts (selects) only a combination of the electric conduction time t and the applied voltage V corresponding to a point on a line that is in the straight line L and delimited by the range $V_{Range}$ on the horizontal line (the applied voltage) (for example, $S_2$, $S_3$, $S_4$), as set values usable for driving the atomizing unit 104 by the electric energy $W_1$, wherein the straight line L is that determined in step S302 based on the electric energy ($W_1$) to be supplied to the heater.

Next, in step S308, the control unit 130 drives the heater of the atomizing unit 104 by use of the combination of the electric conduction time t and the applied voltage V selected as explained above. As a result thereof, the atomizing unit 104 can be controlled by use of an optimum combination of the electric conduction time t and the applied voltage V.

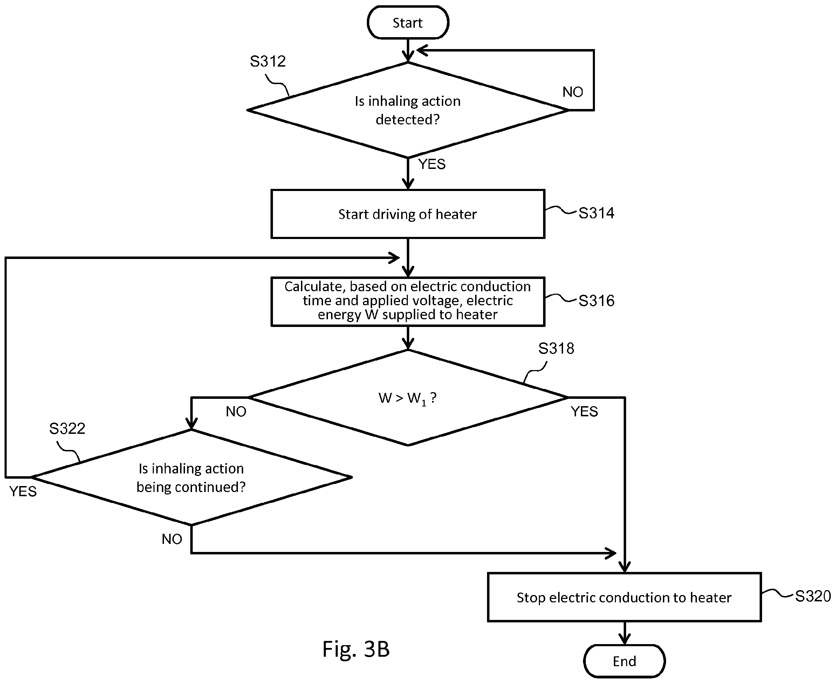

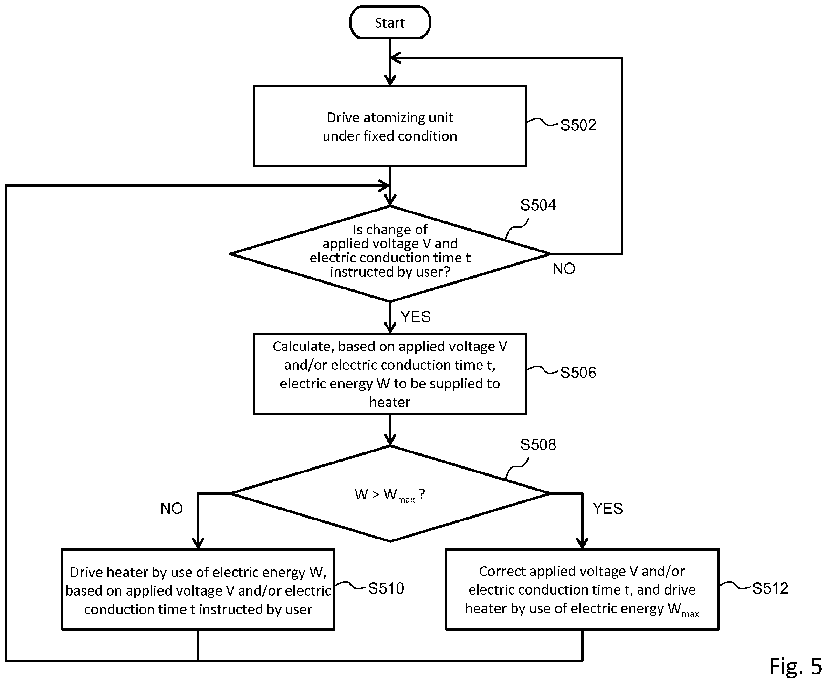

Fig. 3B is a flow chart showing an example of control for driving, based on electric conduction time t and an applied voltage V, the heater for the atomizing unit 104. Control according to the flow chart represents, in a more tangible manner, processing in step S308 in the flow chart in Fig. 3A, for example. Further, the above control according to the flow chart can be applied to control for driving the heater in step S510 and step S512 in the flow chart in Fig. 5 (control in a second mode) which will be explained later.

First, in step S312, the control unit 130 judges, based on an output from the puff sensor 122, whether or not an inhaling action of a user is detected. If an inhaling action of a user is detected, the process proceeds to step S314, and, if not, step S312 is repeated.

In the case that an inhaling action of a user is detected, the control unit 130 starts supplying of the applied voltage V (for example, the applied voltage selected in step S306 in the flow chart in Fig. 3A) to the heater, and starts counting of electric conduction time thereof, in step S314.

Next, in step S316, the control unit 130 calculates, based on the counted value $t_c$ of the electric conduction time and the voltage V applied to the heater, the electric energy supplied to the heater until that point in time, i.e., $W=(V^2/R)*t_c$.

Next, in step S 318, the control unit 130 judges whether or not the electric energy W, that is calculated in step S316 and represents the quantity actually supplied to the heater until the present point in time, exceeds the electric energy supply $W_1$ to the heater (for example, the electric energy determined in step S302 in the flow chart in Fig. 3A) that is calculated based on the electric conduction time t (for example, the electric conduction time selected in step S306 in the flow chart in Fig. 3A) and the applied voltage V. Note that the control unit 130 may be constructed to judge whether or not the counted value $t_c$ of the conduction time has reached the conduction time t, instead of performing the above judgment (these two judging methods are equivalent to each other).

If it is judged as a result of judgment in step S318 that the electric energy W actually supplied to the heater exceeds $W_1$ (in the case that the counted value $t_c$ of electric conduction time has reached the electric conduction time t), the control unit 130 stops electric conduction to the heater in step S320. As a result thereof, an appropriate quantity of aerosol, that is determined based on the electric conduction time t and the applied voltage V, is delivered to the user.

On the other hand, if it is judged as a result of judgment in step S318 that the electric energy W actually supplied to the heater does not exceed $W_1$ (in the case that the counted value $t_c$ of electric conduction time has not yet reached the electric conduction time t), the control unit 130 judges in step S322, based on an output from the puff sensor 122, whether or not an inhaling action of the user is being continued. In the case that an inhaling action of the user is being continued, the process returns to step S316. In the case that an inhaling action of the user has been discontinued, the process proceeds to step S320, and the control unit 130 stops electric conduction to the heater. Thus, in the case that the user has stopped an inhaling action before completion of generation of a desired quantity of aerosol in the atomizing unit 104, generation of aerosol is stopped as intended by the user.

< Control in a second mode >

Fig. 5 is a flow chart showing operation of the control unit 130 relating to control in a second mode. As explained above, the control in the second mode is an example of control in the case that one or both of the applied voltage V and the electric conduction time t relating to the heater for the atomizing unit 104 is/are directly set via the user setting unit 150, and is applicable to both the control for the first atomizing unit 104A and the control for the second atomizing unit 104B without discrimination.

First, in step S502, the control unit 130 drives the atomizing unit 104 (the first atomizing unit 104A or the second atomizing unit 104B) under a predetermined fixed condition. Step S502 represents an initial state of operation of the control unit 130. For example, an initial set value $W_0$ of electric energy, that should be supplied to the heater for the atomizing unit 104 when starting operation of the flavor inhaler 100, is stored in the memory 140 in advance. When user has started use of the flavor inhaler 100 (has started inhaling action), the control unit 130 reads the initial set value of electric energy supplied to the heater out of the memory 130, and drives the atomizing unit 104 according to the read value of the electric energy.

Next, in step S504, the control unit 130 judges whether or not a user instruction for changing at least one of the applied voltage V and the electric conduction time t relating to the heater for the atomizing unit 104 is inputted via the user setting unit 150. If such a user instruction is inputted, the process proceeds to step S506, and, if not, the process returns to step S502.

In step S506, the control unit 130 calculates, based on the user instruction inputted via the user setting unit 150 and in accordance with the relational formula of electric energy $W=(V^2/R)*t$, a changed electric energy W to be supplied to the heater for the atomizing unit 104. Note that, in the case that one of the applied voltage V and the electric conduction time t relating to the heater for the atomizing unit 104 only (for example, an applied voltage) is inputted as the user instruction in step S504, another (for example, electric conduction time) is set in such a manner that a set value presently used for driving the heater is used as the value thereof.

Next, in step S508, the control unit 130 judges whether or not the electric energy W, which is calculated in step S506 and to be supplied to the heater, exceeds the predetermined upper limit value $W_{max}$. The upper limit value $W_{max}$ represents the maximum electric energy allowed to be supplied to the heater for the atomizing unit 104. For example, the upper limit value $W_{max}$ is defined as the electric energy such as that the consumption rate of the aerosol source increases and the aerosol tends to be easily exhausted as a result thereof, or a substance which is not desired to be generated may be generated from the aerosol source, due to overheating of the heater when electric energy having a value that exceeds the value of the upper limit value $W_{max}$ is supplied to the heater. Alternatively, the upper limit value $W_{max}$ may be the electric energy (for example, the maximum value of the output of the driving circuit) determined based on restriction relating to the driving circuit which supplies electric power to the heater. Note that each of the upper limit values of the variable ranges of the aerosol generation quantities u and v of the first atomizing unit 104A and the second atomizing unit 104B, that are explained above, is the value determined based on the upper limit value $W_{max}$ of the electric energy supplied to the heater. If the calculated value W of the electric energy exceeds the upper limit value $W_{max}$, the process proceeds to step S512, and, if not, the process proceeds to step S510.

In step S510, the control unit 130 drives the heater for the atomizing unit 104, in accordance with the applied voltage V and the electric conduction time t relating to the user instruction relating to step S504. The electric energy supplied to the heater at that time is electric energy that is calculated in step S506, thus, electric energy equal to or lower that the upper limit value $W_{max}$. In this manner, in response to the user instruction to the user setting unit 150 for designating at least one of the applied voltage and the electric conduction time relating to the heater, the quantity of aerosol generated in the atomizing unit 104 can be changed.

In step S512, the control unit 130 corrects the applied voltage V and/or the electric conduction time t relating to the user instruction relating to step S504 so as to make the electric energy supled to the heater for the atomizing unit 104 to be equal to the upper limit value $W_{max}$, and drives the heater for the atomizing unit 104 in accordance with the corrected applied voltage and/or the corrected electric conduction time. Thus, even in the case that the user instruction inputted via the user setting unit 150 is that for instructing supply of electric energy having a value larger than the upper limit value $W_{max}$ to the heater, the heater for the atomizing unit 104 is driven by use of electric energy having a value equal to the upper limit value $W_{max}$, so that it is possible to prevent supply of excessive electric energy to the heater. Note that it may be possible to construct the control unit 130 in such a manned that it notifies a use of an error for indicating that the electric energy value W calculated in step S506 exceeds the upper limit value $W_{max}$, and prompts the user to repeat step S504 to input appropriate values for the applied voltage V and the electric conduction time t.

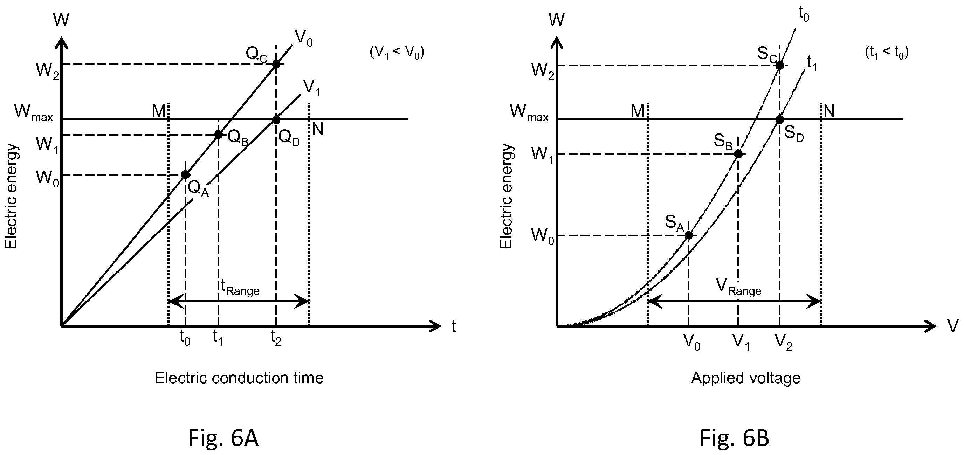

Fig. 6A is a figure for explaining the electric energy supplied to the heater for the atomizing unit 104, in the case that electric conduction time t is designated by a user via the user setting unit 150. For simplifying explanation, it is supposed that the user changes, by using a user instruction to the user setting unit 150, the electric conduction time t for the heater only, and does not change the applied voltage V. In an initial state, the heater for the atomizing unit 104 is driven based on the applied voltage $V_0$ and the electric conduction time $t_0$, for making the electric energy $W_0$ to be supplied to the heater (point $Q_A$). In the case that the user has made the electric conduction time to be increased to $t_1$ via the user setting unit 150, and that the electric energy value $W_1$ calculated in step S506 at that time is equal to or less than the upper limit value $W_{max}$, the heater for the atomizing unit 104 is driven, as explained in relation to step S510, based on the applied voltage $V_0$ that has not changed and the elec-

tric conduction time $t_1$ that is instructed by the user, so that the electric energy W supplied to the heater becomes $W_1$ (point $Q_B$). It is supposed that the user has made the electric conduction time to be increased further to $t_2$ via the user setting unit 150, and, as a result thereof, the electric energy value $W_2$ calculated in step S506 exceeds the upper limit value $W_{max}$ (point $Q_C$). Then, as explained in relation to step S512, the control unit 130 corrects the applied voltage and the electric conduction time relating to the heater in such a manner that the value of the electric energy supplied to the heater becomes equal to the upper limit value $W_{max}$. For example, the control unit 130 adopts the electric conduction time $t_2$ instructed by the user as it stands, and reduces the applied voltage to $V_1$ (point $Q_D$). The control unit 130 drives the heater for the atomizing unit 104 based on the applied voltage to $V_1$ and the electric conduction time $t_2$ that correspond to the point $Q_D$. In the case that the electric conduction time t relating to the heater for the atomizing unit 104 is directly set via the user setting unit 150 in this manner, the heater can be driven in such a manner that the value of the electric energy supplied to the heater does not exceed the upper limit value $W_{max}$.

Fig. 6B is a figure for explaining the electric energy supplied to the heater for the atomizing unit 104, in the case that an applied voltage V is designated by a user via the user setting unit 150. Similar to the case of Fig. 6A, for simplifying explanation, it is supposed that the user does not change the electric conduction time t. In an initial state, the heater for the atomizing unit 104 is driven based on the applied voltage $V_0$ and the electric conduction time $t_0$, for making the electric energy $W_0$ to be supplied to the heater (point $S_A$). In the case that the user has made, via the user setting unit 150, the applied voltage to be increased to $V_1$, and that the heater for the atomizing unit 104 is driven based on the applied voltage $V_1$ and the electric conduction time $t_0$, the electric energy supplied to the heater becomes $W_1$ (point $S_B$). In the case that the user has made, via the user setting unit 150, the applied voltage to be increased further to $V_2$, the electric energy value $W_2$ calculated in step S506 exceeds the upper limit value $W_{max}$ (point $S_C$) as a result thereof. The control unit 130 drives the heater for the atomizing unit 104 based on the applied voltage $V_2$ that relates to the user instruction and the electric conduction time $t_1$ that is shorter than the initial electric conduction time $t_0$, for making the value of the electric energy supplied to the heater becomes equal to the upper limit value $W_{max}$ (point $S_D$). In the case that the applied voltage V relating to the heater for the atomizing unit 104 is directly set via the user setting unit 150 in this manner, the heater can be driven in such a manner that the value of the electric energy supplied to the heater does not exceed the upper limit value $W_{max}$.

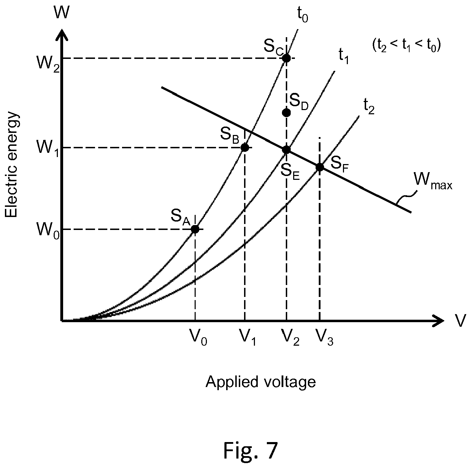

Fig. 7 is a figure for explaining a modified example of control performed in the case that the applied voltage V is designated by a user via the user setting unit 150. In this example, control similar to that relating to Fig.

6B is also performed until the calculated value of the electric energy supplied to the heater exceeds the upper limit value $W_{max}$ (point $S_A$ and point $S_B$). However, as shown in Fig. 7, the upper limit value $W_{max}$ in this example is set in such a manner that it decreases as the applied voltage V increases. In the case that the user has made, via the user setting unit 150, the applied voltage to be increased to $V_2$, and that the electric energy value $W_2$ calculated in step S506 exceeds the upper limit value $W_{max}$ (point $S_C$) as a result thereof, the control unit 130 makes the electric energy supplied to the heater to be reduced to an upper limit value $W_{max}$ (point $S_E$) that is smaller than the value of the electric energy corresponding to the point $S_D$ in Fig. 6B. In the case that the user has made, via the user setting unit 150, the applied voltage to be increased further to $V_3$, the control unit 130 makes the electric energy supplied to the heater to be reduced to a smaller upper limit value $W_{max}$ (point $S_F$) that is smaller than the value of the electric energy corresponding to the point $S_E$ in Fig. 6B. In this manner, the control unit 130 gradually reduces the electric energy supplied to the heater as the applied voltage V increases. Since the quantity of heat generated by the heater per unit time becomes large as the applied voltage V becomes large, overheating can be prevented by gradually reducing the electric energy supplied to the heater as the applied voltage V increases.

Note that control based on the maximum electric energy $W_{max}$, that is shown in each of Figs. 6A, 6B, and 7, can also be applied, in a similar manner, to the control in the above explained first mode. That is, the control unit 130 may be constructed in such a manner that, in the case that the aerosol generation quantity u and/or the aerosol generation quantity v determined in step S206, S208, or S210 in Fig. 2 exceed/exceeds the maximum aerosol quantity that can be generated by the atomizing units/unit 104, the control unit 130 controls the atomizing units/unit 104 in such a manner that the quantities/quantity of electric energy supplied to the heaters/heater for the atomizing units/unit 104 are/is limited to the upper limit values/value $W_{max}$ such as those/that shown in Fig. 6A, 6B, or 7.

< Control in a third mode >

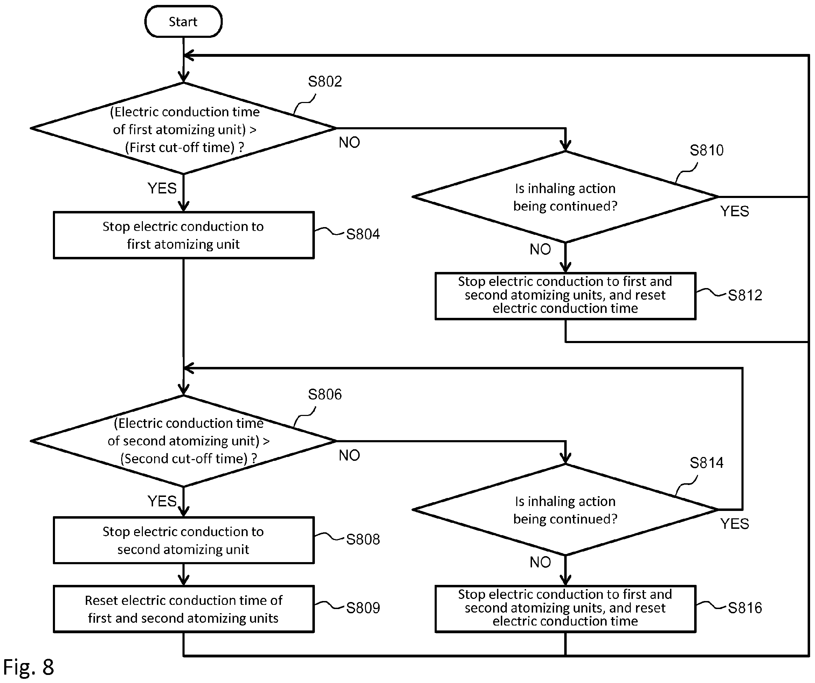

Fig. 8 is a flow chart showing operation of the control unit 130 relating to control in a third mode. The control in the third mode is an example of control for stopping electric conduction to the atomizing unit 140 in response to time of electric conduction to the atomizing unit 104 in a single atomizing action.

First, in step S802, the control unit 130 judges whether or not time of electric conduction to the first atomizing unit 104A in a single atomizing action exceeds first cutoff time. If the time of electric conduction to the first atomizing unit 104A in the single atomizing action exceeds the first cutoff time, the process proceeds to step S804, and, if not, the process proceeds to step S810.

In step S804, the control unit 130 stops electric conduction from the battery 114 to the first atomizing unit 104A. Thus, in the case that a single inhaling action performed by a user is continued for a time period longer than a predetermined time period (the first cutoff time), generation of aerosol by the first atomizing unit 104A is stopped, and generation of the flavor component in the flavor source 106 is also stopped accordingly. Thus, it is possible to prevent supply of an excessive quantity of the flavor component above the quantity equal to a predetermined flavor component quantity (the flavor component quantity q set via the user setting unit 150) to the user during a single inhaling action performed by the user.

Next, in step S806, the control unit 130 judges whether or not time of electric conduction to the second atomizing unit 104B in a single atomizing action exceeds second cutoff time. The second cutoff time is a length of time that is set in such a manner that the quantity of aerosol generated when electric conduction to the first atomizing unit 104A is continued for the first cutoff time is different from the quantity of aerosol generated when electric conduction to the second atomizing unit 104B is continued for the second cutoff time. For example, the length of the second cutoff time is different from that of the first cutoff time. In a different example, the length of the second cutoff time is longer than that of the first cutoff time. The electric energy that has supplied to the heater until the second cutoff time has elapsed corresponds to the above explained upper limit value $W_{max}$. If the time of electric conduction to the second atomizing unit 104B in the single atomizing action exceeds the second cutoff time, the process proceeds to step S808, and, if not, the process proceeds to step S814.

In step S808, the control unit 130 stops electric conduction from the battery 114 to the second atomizing unit 104B. Thus, in the case that a single inhaling action performed by a user is continued for a time period longer than a predetermined time period (the second cutoff time), generation of aerosol by the second atomizing unit 104B is stopped, in addition that generation of aerosol by the first atomizing unit 104A is stopped. Next, in step S809, the control unit 130 resets the electric conduction time relating to the first atomizing unit 104A and the second atomizing unit 104B, and, thereafter, the process returns to step S802.

In step S810, the control unit 130 judges, based on an output from the puff sensor 122, whether or not the single inhaling action by the user is being continued. If the single inhaling action is being continued, the process returns to step S802 for repeating judgment relating to the first cutoff time, and, if the single inhaling action has been completed, the process proceeds to step S812.

In step 812, the control unit 130 stops electric conduction from the battery 114 to the first atomizing unit 104A and the second atomizing unit 104B, and, at the same time, resets the electric conduction time relating to the first atomizing unit 104A and the second atomizing unit 104B, and, thereafter, the process returns to step S802.

In step S814, the control unit 130 judges, based on an output from the puff sensor 122, whether or not the single inhaling action by the user is being continued. If the single inhaling action is being continued, the process returns to step S806 for repeating judgment relating to the second cutoff time, and, if the single inhaling action has been completed, the process proceeds to step S816.

In step S816, the control unit 130 stops electric conduction from the battery 114 to the first atomizing unit 104A and the second atomizing unit 104B, and, at the same time, resets the electric conduction time relating to the first atomizing unit 104A and the second atomizing unit 104B, and, thereafter, the process returns to step S802.

Thus, for example, in the case that a single inhaling action is continued by a user, intentionally or without intention, for a time period longer than a predetermined time period, electric conduction to the atomizing units 104 is forcedly stopped by the control in the third mode. Accordingly, it is possible to prevent the flavor component quantity q and the total aerosol quantity p delivered to the user from excessing the set values inputted via the user setting unit 150, and, at the same time, prevent the heaters for the atomizing units 104 from overheating thereof, thus generation of a substance which is not desired to be generated in the aerosol source can be suppressed.

< Control in a fourth mode >

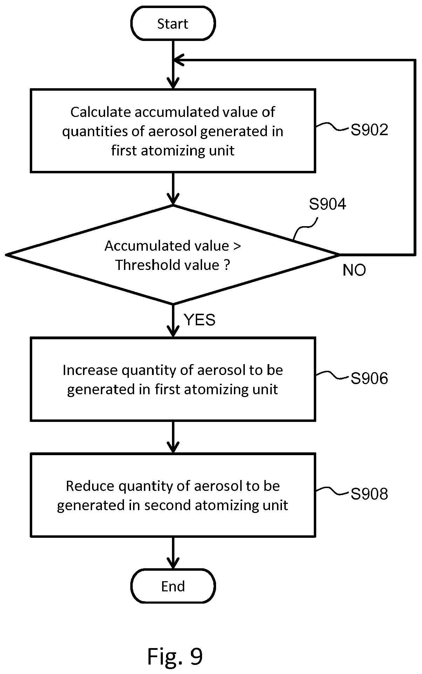

Fig. 9 is a flow chart showing operation of the control unit 130 relating to control in a fourth mode. The control in the fourth mode is an example of control of the first atomizing unit 104A and/or the second atomizing unit 104B that is performed based on an accumulated value of quantities of aerosol generated in the first atomizing unit 104A.

First, in step S902, the control unit 130 calculates an accumulated value of quantities of aerosol generated in the first atomizing unit 104A. Usually, the quantity of aerosol to be generated is determined based on energy supplied to the aerosol source. For example, data representing relationship between the electric energy supplied to the heater for the first atomizing unit 104A and the quantities of aerosol generated from the aerosol source when the heater is heated by the electric energy is stored in the memory 140 in advance. The control unit 130 observes electric energy (= (electric power) * (electric conduction time)) supplied from the battery 114 to the heater for the first atomizing unit 104A over time, refers to the aerosol generation quantity characteristic data, and successively obtains values of the aerosol generation quantities corresponding to respective observed values from the memory 140 and adds them, for thereby obtaining an accumulated value of quantities of aerosol generated in the first atomizing unit 104A in an inferential manner. Under the condition that the electric power supplied to the heater per unit time is constant, it may be constructed in such a manner that the control unit 130 observes time of electric conduction to the heater instead of observing electric energy supplied to the heater, and obtains an accumulated value of quantities of generated aerosol based on an accumulated value of electric conduction time. Note that the accumulated value of quantities of aerosol may be an accumulated value in a single inhaling period, or an accumulated value of aerosol quantities with respect to respective inhaling periods over plural inhaling periods.

Next, in step S904, the control unit 130 judges whether or not the accumulated value of the quantities of aerosol generated in the first atomizing unit 104A exceeds a threshold value. If the accumulated value of the quantities of generated aerosol exceeds the threshold value, the process proceeds to step S906, and, if not, the process returns to step S902. The judgment in step S904 may be performed at any of timing 1) after a single atomizing action is completed, 2) during a predetermined time lag that is between a point in time when an inhaling action by a user is detected by the puff sensor 122 and a point in time when atomizing of aerosol is started, or 3) during an atomizing action (during a period of electric conduction to the heater), for example.

In step S906, the control unit 130 drives the first atomizing unit 104A in such a manner that the quantity of aerosol generated in the first atomizing unit 104A is to be increased. Specifically, the control unit 130 decides the value of electric energy supplied to the heater for the first atomizing unit 104A to be that larger than the present value, and drives the first atomizing unit 104A in such a manner that the decided electric energy is supplied to the heater. There may be a case that the ability of the flavor source 106 to emit flavor components is deteriorated gradually due to flow of aerosol; however, by performing the control in step S906, reduction in the quantity of flavor components emitted from the flavor source 106 can be compensated. Thus, in the flavor inhaler 100, effect due to consumption in the flavor source can be suppressed, and a constant quantity of flavor can be supplied to the user.

Next, in step S908, the control unit 130 drives the second atomizing unit 104B in such a manner that the quantity of aerosol generated in the second atomizing unit 104B is to be decreased. Specifically, the control unit 130 decides the value of electric energy supplied to the heater for the second atomizing unit 104B to be that smaller than the present value, and drives the second atomizing unit 104B in such a manner that the decided electric energy is supplied to the heater. As explained above, the quantity of flavor components supplied to the user is maintained to be constant, in the control in step S906; on the other hand, as a result that the quantity of aerosol generated in the first atomizing unit 104A increases, the total quantity of aerosol supplied to the user increases at the same time, without any relationship with user's intention. However, by performing the control in step S908, the quantity of aerosol from the second atomizing unit 104B is reduced in such a manner that the quantity of aerosol from the first atomizing unit 104A is to be cancelled out thereby. Thus, in the flavor inhaler 100, both the quantity of flavor components and the total quantity of aerosol supplied to the user can be maintained to be constant.

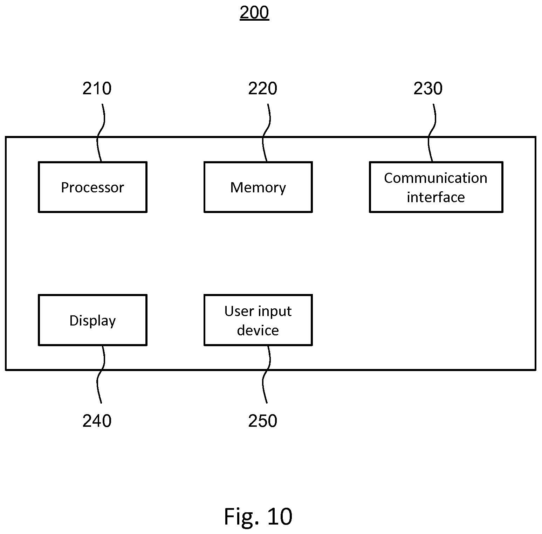

Fig. 10 is a block diagram showing a construction of an external device 200 which can be used for allowing a user to set an operation mode of the flavor inhaler 100 and confirm an operation state thereof. For example, the external device 200 may be connected for communication with a smart phone, a tablet terminal, a PC (a personal computer), or the flavor inhaler 100, and realized as another electronic device which has any form and has ability to electronically process information relating to operation of the flavor inhaler 100. The external device 200 comprises a processor 210, a memory 220, a communication interface 230, a display 240, a user input device 250, and other appropriate optional components (not shown in the figure). The processor 210 reads computer executable instructions stored in the memory 220 and executes them, for operating the external device 200 in accordance with them. The communication interface 230 establishes communication connection with the user setting unit 150, which functions as a communication interface of the flavor inhaler 100, for allowing mutual communication between the external device 200 and the flavor inhaler 100. For communication connection between the communication interface 230 in the external device 200 and the user setting unit 150 (a communication interface) in the flavor inhaler 100, wired connection such as USB connection or the like or wireless connection such as a wireless LAN, Bluetooth (a registered trademark), infrared communication, or the like is usable, for example. The display 240 presents visual information, that relates to operation of the external device 200, to a user. The user input device 250 accepts input manipulation that is applied to the external device 200 by a user. The display 240 and the user input device 250 may be constructed, in an integrated manner, as a touch panel.

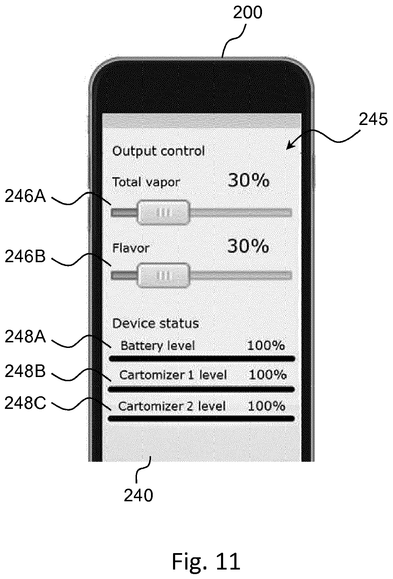

Fig. 11 shows an example of a user interface screen 245 displayed on the display 240 in the external device 200. A user of the flavor inhaler 200 may use the user interface screen 245 displayed on the display 240 in the external device 200, for setting operation with respect to the flavor inhaler 100. Also, the user may be able to know a state of operation of the flavor inhaler 100 via the user interface screen 245.

As shown in Fig. 11, the user interface screen 245 comprises a first slider 246A and a second slider 246B. The first slider 246A is an operating element which allows a user to set or adjust the total aerosol quantity p with respect to the flavor inhaler 100. The second slider 246B is an operating element which allows a user to set or adjust the flavor component quantity q with respect to the flavor inhaler 100. For example, a user inputs a user instruction with respect to the flavor inhaler 100 by maintaining them in such a manner that the positions of the first slider 246A and the second slider 246B are maintained at those corresponding to a desired total aerosol quantity p and a desired flavor component quantity q, respectively, or the first slider 246A and the second slider 246B are slid to the above positions, respectively. The processor 210 specifies, based on the positions of the first slider 246A and the second slider 246B, the user instruction from the user, and provides the user setting unit 150 in the flavor inhaler 100, via the communication interface 230, with the specified user instruction. The control unit 130 in the flavor inhaler 100 uses the thus obtained user instruction (i.e., the total aerosol quantity p and the flavor component quantity q) for controlling, in accordance with the above explained flow chart in Fig. 2 (the control in the first mode), the aerosol generation quantity u of the first atomizing unit 104A and the aerosol generation quantity v of the second atomizing unit 104B. Accordingly, the user can set or adjust both the total aerosol quantity p and the flavor component quantity q with respect to the flavor inhaler 100 to preferred quantities, by manipulating the user interface screen 245 in the external device 200.

Note that it may be possible to construct the user interface screen 245 in such a manner that only one of the first slider 246A and the second slider 246B is included therein. In such a case, a user can set or adjust only one of the total aerosol quantity p and the flavor component quantity q with respect to the flavor inhaler 100 to a desired quantity by manipulating the user interface screen 245. In the case that the total aerosol quantity p is set or adjusted to a quantity desired by a user, the flavor component quantity q may be maintained to be constant, or increased/decreased to correspond to the total aerosol quantity p. In the case that the flavor component quantity q is set or adjusted to a quantity desired by a user, the process relating thereof is similar to that in the above case. Further, it may be possible to use two sliders for directly designating the aerosol generation quantity u of the first atomizing unit 104A and the aerosol generation quantity v of the second atomizing unit 104B, respectively, in place of the above explained first slider 246A and second slider 246B. Further, an operating element different from the operating element having the form of a slider, for example, an operating element having a form comprising a "+ button" and a "-button" for increasing/decreasing a numerical value, may be adopted.

The user interface screen 245 shown in Fig. 11 further comprises a first status bar 248A, a second status bar 248B, and a third status bar 248C. The first status bar 248A shows a remaining battery capacity of the battery 114 in the flavor inhaler 100. The second status bar 248B and the third status bar 248C show degrees representing remaining quantities of aerosol sources in the first reservoir 102A and the second reservoir 102B in the flavor inhaler 100, respectively. For example, the control unit 130 in the flavor inhaler 100 detects, in a periodic manner, the remaining battery capacity of the battery 114 and the degrees representing remaining quantities of aerosol sources in the first reservoir 102A and the second reservoir 102B, and provides the external device 200 with information thereof, via communication connection with the external device 200. The processor 210 in the external device 200 reflects the above information obtained from the flavor inhaler 100 in the respective status bars 248A, 248B, and 248C in the user interface screen 245. Accordingly, a user can know the state of operation of the flavor inhaler 100, by referring to the respective status bars 248A, 248B, and 248C in the user interface screen 245. It may be possible to display, on the user interface screen 245, other status information relating to the flavor inhaler 100 (for example, the number of times of inhaling actions performed by a user, the accumulated inhaled quantities of the aerosol and the flavor components, and so on).

In another embodiment, the user interface screen 245 may comprise a third slider, which is not shown in the figure, for designating a ratio r between the total aerosol quantity p and the flavor component quantity q (for example, $r=q/p$). For example, the user interface screen 245 may be constructed to include two sliders such as the first slier 246A and the third slider. In such a case, a user instruction including the total aerosol quantity p and the ratio r is given from the external device 200 to the user setting unit 150 in the flavor inhaler 100. The control unit 130 in the flavor inhaler 100 can specify the flavor component quantity q based on the above user instruction, and control thereby the aerosol generation quantity u of the first atomizing unit 104A and the aerosol generation quantity v of the second atomizing unit 104B, in a manner similar to that in the above case. Similarly, the user interface screen 245 may be constructed to include two sliders such as the second slier 246B and the third slider, and a user instruction including the flavor component quantity q and the ratio r may be given to the user setting unit 150 in the flavor inhaler 100.

Further, the user interface screen 245 may be constructed to include three sliders such as the first slider 246A, the second slier 246B, and the third slider. In such a construction, it may be possible to additionally provide an instruction from a user for fixing one of the total aerosol quantity p, the flavor component quantity q, and the ratio r to a constant value. For example, a user inputs, into the user interface screen 245, an instruction for fixing the flavor component quantity q to the maximum value (by checking a check box, which is not shown in the figure and may be formed at a side part of the second slider 246B, for example). In such a case, the second slider 246B is shown as that in a state that it is not allowed to be slid, and a user can manipulate the first slider 246A and the third slider only. Thereafter, for example, in the case that the user has slid the third slide for adjusting the ratio r to a desired value, the processor 210 in the external device 200 may specify the total aerosol quantity p based on the flavor component quantity q (the maximum value) and the ratio r (the value instructed by the third slider), and automatically move the first slider 246A to a position corresponding to the specified value. In this example, the user instruction may be constructed in such a manner that it includes the total aerosol quantity p specified by the processor 210, in addition to the flavor component quantity q and the ratio r directly specified by the user. As a result that the above user instruction is given from the external device 200 to the user setting unit 150 in the flavor inhaler 100, the control unit 130 in the flavor inhaler 100 can control, in a manner similar to that in the above case, the aerosol generation quantity u of the first atomizing unit 104A and the aerosol generation quantity v of the second atomizing unit 104B.

In another embodiment, the user interface screen 245 may comprise a display for notifying the matter that a value of the total aerosol quantity p or the flavor component quantity q inputted by a user is inappropriate, when such a matter has occurred. For example, as explained above, the processor 210 in the external device 200 provides the flavor inhaler 100 with a user instruction (the total aerosol quantity p and the flavor component quantity q) inputted via the user interface screen 245, and the control unit 130 determines, based on the above user instruction, the aerosol generation quantity u of the first atomizing unit 104A and the aerosol generation quantity v of the second atomizing unit 104B (refer to step S206, step S208, and step S210 in the control in the first mode). The control unit 130 in the flavor inhaler 100 judges whether or not the obtained aerosol quantity u and the obtained aerosol quantity v exceed the maximum aerosol quantities (for example, the maximum aerosol quantity that is determined based on the heating capacity of a heater, electric power that can be supplied from a battery, and so on) that can be supplied by the respective atomizing units 104A and 104B, and, if one or both of the aerosol quantities u and v exceeds/exceed the maximum value/values, instructs the external device 200 of displaying an error notification for notifying that the user instruction is inappropriate. The error notification may be that including an instruction requesting the user of re-inputting a different total aerosol quantity p and a different flavor component quantity q again.

In a further different embodiment, the first slider 246A and the second slider 246B in the user interface screen 245 may be constructed in such a manner that the ranges that the sliders can be slid are limited for preventing inputting of an inappropriate total aerosol quantity p and an inappropriate flavor component quantity q, respectively. For example, as explained above, the upper limit value of each of the variable ranges of the aerosol generation quantities u and v with respect to the first atomizing unit 104A and the second atomizing unit 104B is defined based on the maximum electric energy $W_{max}$ that can be supplied to the heater of each of the atomizing units 104. The control unit 130 in the flavor inhaler 100 can calculate, by use of the upper limit values of the aerosol generation quantities u and v and based on the formulas (1) and (2) shown above, the maximum values that the total aerosol quantity p and the flavor component quantity q can take. The control unit 130 provides the external device 200 with the calculated maximum values of the total aerosol quantity p and the flavor component quantity q, and the processor 210 in the external device 200 displays, on the display 240, the user interface screen 245 including the first slider 246A and the second slider 246B having forms that do not allow movement to positions above those corresponding to their maximum values. Thus, it becomes possible to prevent erroneous input of an inappropriate total aerosol quantity p and an inappropriate flavor component quantity q (for example, those beyond the ability of the atomizing units 104) by a user from occurring. Note that the lower limit values of the variable ranges of the respective sliders may be set in a manner similar to that in the above case.

Although the embodiments of the present invention have been explained in the above description, the present invention is not limited to the embodiments, and the embodiments can be modified in various ways without departing from the scope of the gist of the present invention.

REFERENCE SIGNS LIST

| | |
|---|---|
| 100 | Flavor inhaler |
| 102A | First reservoir |
| 102B | Second reservoir |
| 104A | First atomizing unit |
| 104B | Second atomizing unit |
| 106 | Flavor source |
| 108 | Mouthpiece member |
| 110A | First aerosol flow path |
| 110B | Second aerosol flow path |
| 114 | Battery |
| 116 | Air intake channel |
| 118 | Mixing chamber |
| 122 | Puff sensor |
| 130 | Control unit |
| 140 | Memory |
| 150 | User setting unit |
| 200 | External device |
| 210 | Processor |
| 220 | Memory |
| 230 | Communication interface |
| 240 | Display |
| 245 | User interface screen |
| 246A | First slider |
| 246B | Second slider |
| 248A | First status bar |
| 248B | Second status bar |
| 248C | Third status bar |
| 250 | User input device |

**Claims**

1. A flavor inhaler comprising:

   a mouthpiece;
   a first atomizing unit for atomizing an aerosol source for generating aerosol;
   a flavor source positioned between the first atomizing unit and the mouthpiece;
   a first flow path constructed for guiding the aerosol generated in the first atomizing unit to the mouthpiece through the flavor source;
   a second atomizing unit for atomizing an aerosol source for generating aerosol;
   a second flow path constructed for guiding the aerosol generated in the second atomizing unit to the mouthpiece without passing through the flavor source; and
   a control unit which can change the quantity of aerosol to be generated in at least one of the first atomizing unit and the second atomizing unit.

2. The flavor inhaler according to Claim 1, wherein the control unit controls, in an independent manner, the quantity of aerosol to be generated in the first atomizing unit and the quantity of aerosol to be generated in the second atomizing unit.

3. The flavor inhaler according to Claim 1 or 2, wherein the flavor source adds a quantity of flavor components, that corresponds to the quantity of aerosol generated in the first atomizing unit, to the aerosol passing through the flavor source, and the control unit controls the quantity of aerosol to be generated in the first atomizing unit in such a manner that a predetermined quantity of the flavor components is delivered to the mouthpiece.

4. The flavor inhaler according to one of Claims 1 to 3, wherein the control unit controls, in response to a set value for setting at least one of a total quantity of aerosol and a quantity of flavor components which should be delivered to the mouthpiece, the quantities of aerosol to be generated in the first atomizing unit and the second atomizing unit.

5. The flavor inhaler according to Claim 4 further comprising a user setting unit for receiving a user instruction relating to the setting value.

6. The flavor inhaler according to Claim 5, wherein the user setting unit is able to communicate with an external device, and is constructed to receive the user instruction via a user interface screen displayed on a display unit in the external device.

7. The flavor inhaler according to Claim 6, wherein the user setting unit is constructed to receive the user instruction relating to both the total quantity of aerosol and the quantity of flavor components via a single user interface screen displayed on a display unit in the external device.

8. The flavor inhaler according to Claim 6, wherein the user setting unit is constructed to receive the user instruction relating a ratio between the total quantity of aerosol and the quantity of flavor components via the user interface screen.

9. The flavor inhaler according to Claim 6, wherein the control unit makes the user interface screen to give notice of an error, in the case that the quantity of aerosol to be generated, based on the user instruction given via the user interface screen, in at least one of the first atomizing unit and the second atomizing unit exceeds a predetermined maximum value.

10. The flavor inhaler according to Claim 6, wherein the control unit determines, based on a variable range of the quantity of aerosol to be generated in the first atomizing unit and a variable range of the quantity of aerosol to be generated in the second atomizing unit, a variable range of the total quantity of aerosol and a variable range of the quantity of flavor components, and supplies the values representing the variable range of the total quantity of aerosol and the variable range of the quantity of flavor components to the external device for displaying them in the user interface screen.

11. The flavor inhaler according to Claim 6, wherein the control unit stops generation of the aerosol in the first atomizing unit or the second atomizing unit, in the case that the user instruction given via the user interface screen satisfies a predetermined condition.

12. The flavor inhaler according to one of Claims 1 to 11, wherein
the first atomizing unit and the second atomizing unit are constructed to atomize aerosol by heating by use of heaters, and
a resistance value of a heater for the first atomizing unit is larger than a resistance value of a heater for the second atomizing unit.

13. The flavor inhaler according to one of Claims 1 to 12, wherein
the control unit stops electric conduction to the first atomizing unit when continuous electric conduction time of electric conduction to the first atomizing unit exceeds a first cut-off time, and stops electric conduction to the second atomizing unit when continuous electric conduction time of electric conduction to the second atomizing unit exceeds a second cut-off time, wherein

the quantity of aerosol that is generated when the first atomizing unit is energized for a period of the first cut-off time is different from the quantity of aerosol that is generated when the second atomizing unit is energized for a period of the second cut-off time.

14. The flavor inhaler according to Claim 13, wherein the length of the first cut-off time is different from the length of the second cut-off time.

15. The flavor inhaler according to Claim 14, wherein the length of the first cut-off time is shorter than the length of the second cut-off time.

16. The flavor inhaler according to one of Claims 1 to 15, wherein the control unit changes the quantity of aerosol to be generated in at least one of the first atomizing unit and the second atomizing unit, by changing one of: electric power to be supplied to the respective atomizing units, and electric conduction time for the respective atomizing units.

17. The flavor inhaler according to one of Claims 1 to 15, wherein the control unit changes the quantity of aerosol to be generated in at least one of the first atomizing unit and the second atomizing unit, by changing both of: electric power to be supplied to the respective atomizing units, and electric conduction time for the respective atomizing units.

18. The flavor inhaler according to Claim 17, wherein the control unit determines the quantity of aerosol to be generated in at least one of the first atomizing unit and the second atomizing unit; determines, based on the determined quantity of aerosol to be generated, the electric energy to be supplied to the corresponding atomizing unit; selects, from plural combinations of a voltage to be applied and electric conduction time for the atomizing unit for obtaining the electric energy, a combination which is covered by a predetermined variable range of electric conduction time or a predetermined variable range of a voltage to be applied; and drives the corresponding atomizing unit with the selected voltage to be applied and the selected electric conduction time.

19. The flavor inhaler according to Claim 16, wherein the control unit determines the quantity of aerosol to be generated in at least one of the first atomizing unit and the second atomizing unit; determines, based on the determined quantity of aerosol to be generated, the electric energy to be supplied to the corresponding atomizing unit; determines, based on a predetermined fixed electric conduction time, a voltage to be applied to the corresponding atomizing unit for obtaining the electric energy; and drives the corresponding atomizing unit with the determined voltage to be applied and the fixed electric conduction time.

20. The flavor inhaler according to Claim 17, wherein the control unit determines a combination of a voltage to be applied and electric conduction time for at least one of the first atomizing unit and the second atomizing unit; calculates, based on the combination, electric energy to be supplied to the corresponding atomizing unit; and, in the case that a calculated value of the electric energy exceeds a predetermined upper limit value, drives the corresponding atomizing unit with a voltage to be applied and electric conduction time that satisfy electric energy of the predetermined upper limit value.

21. The flavor inhaler according to Claim 20, wherein the upper limit value decreases as the voltage to be applied increases.

22. The flavor inhaler according to one of Claims 1 to 15, wherein the control unit can change the quantity of aerosol to be generated in the first atomizing unit by changing the time of electric conduction to the first atomizing unit, and the quantity of aerosol to be generated in the second atomizing unit by changing the electric power to be supplied to the second atomizing unit.

23. The flavor inhaler according to one of Claims 1 to 22, wherein
the control unit can change the quantity of aerosol to be generated in the second atomizing unit; and an upper limit of a variable range of the quantity of aerosol to be generated in the second atomizing unit is larger than the quantity of aerosol to be generated in the first atomizing unit, and a lower limit of the variable range of the quantity of aerosol to be generated in the second atomizing unit is smaller than the quantity of aerosol to be generated in the first atomizing unit.

24. The flavor inhaler according to one of Claims 1 to 23, wherein
the control unit can change the quantity of aerosol to be generated in the first atomizing unit, and a lower limit of the variable range of the quantity of aerosol to be generated in the first atomizing unit is larger than zero.

25. The flavor inhaler according to one of Claims 1 to 22, wherein the variable range of the quantity of aerosol to be generated in the first atomizing unit includes zero.

26. The flavor inhaler according to one of Claims 1 to 25, wherein
the control unit can change both the quantity of aerosol to be generated in the first atomizing unit and the quantity of aerosol to be generated in the second atomizing unit, and
the width of the variable range of the quantity of aerosol to be generated in the first atomizing unit is narrower than the width of the variable range of the quantity of aerosol to be generated in the second atomizing unit.

27. The flavor inhaler according to one of Claims 1 to 26, wherein
the control unit can change both the quantity of aerosol to be generated in the first atomizing unit and the quantity of aerosol to be generated in the second atomizing unit, and
the variable range of the quantity of aerosol to be generated in the first atomizing unit is included in a range between a lower limit value and an upper limit value of the variable range of the quantity of aerosol to be generated in the second atomizing unit.

28. The flavor inhaler according to one of Claims 1 to 27, wherein the control unit determines, based on an accumulated quantity of aerosol passed through the first flow path, the electric energy to be supplied to the first atomizing unit for increasing the quantity of aerosol to be generated in the first atomizing unit.

29. The flavor inhaler according to Claim 28, wherein the control unit determines, based on an accumulated quantity of aerosol passed through the first flow path, the electric energy to be supplied to the second atomizing unit for decreasing the quantity of aerosol to be generated in the second atomizing unit.

30. The flavor inhaler according to Claim 28 or 29, wherein the control unit obtains, based on an accumulated quantity of electric energy supplied to the first atomizing unit, the accumulated quantity of aerosol.

31. The flavor inhaler according to one of Claims 1 to 30 further comprising a mixing chamber for mixing the aerosol flown through the first flow path with the aerosol flown through the second flow path; wherein the mixing chamber is communicated with the mouthpiece.

32. The flavor inhaler according to Claim 31, wherein the mixing chamber has a cross-sectional area larger than any of the cross-sectional areas of the first flow path and the second flow path.

33. The flavor inhaler according to one of Claims 1 to 32, wherein at least one of the first flow path and the second flow path comprises plural flow paths.

34. The flavor inhaler according to one of Claims 1 to 33, wherein the first flow path and the second flow path are arrange in parallel to each other.

Fig. 1

Fig. 2

```
                          ┌─────────┐
                          │  Start  │
                          └────┬────┘
                               │
                               ▼
          ┌──────────────────────────────────────┐
          │ Determine, based on quantity of       │
          │ generated aerosol, quantity of electric│───S302
          │ energy to be supplied to heater        │
          └────────────────┬─────────────────────┘
                           │
                           ▼
          ┌──────────────────────────────────────┐
          │ Specify variable range of electric    │
          │ conduction time or applied voltage     │───S304
          │ with respect to heater                 │
          └────────────────┬─────────────────────┘
                           │
                           ▼
          ┌──────────────────────────────────────┐
          │ Select, based on electric energy       │
          │ supplied to heater, combination of     │
          │ electric conduction time and applied   │───S306
          │ voltage within variable range          │
          └────────────────┬─────────────────────┘
                           │
                           ▼
          ┌──────────────────────────────────────┐
          │ Drive heater by use of selected        │
          │ combination of electric conduction time│───S308
          │ and applied voltage                    │
          └────────────────┬─────────────────────┘
                           │
                           ▼
                      ┌─────────┐
                      │   End   │
                      └─────────┘
```

Fig. 3A

Fig. 3B

Start

S312 — Is inhaling action detected? — NO / YES

S314 — Start driving of heater

S316 — Calculate, based on electric conduction time and applied voltage, electric energy W supplied to heater

S318 — $W > W_1$? — NO / YES

S322 — Is inhaling action being continued? — YES / NO

S320 — Stop electric conduction to heater

End

Fig. 4A

Fig. 4B

EP 3 513 667 A1

```
                              ┌─────────┐
                              │  Start  │
                              └────┬────┘
                                   │              ◄──────────────┐
                                   ▼                             │
                   ┌──────────────────────────────┐             │
                   │      Drive atomizing unit     │  ⌇S502      │
                   │       under fixed condition   │             │
                   └───────────────┬──────────────┘             │
                                   │                  S504       │
      ┌───────────────────────────┤                             │
      │                           ▼                             │
      │                       ╱───────────╲                     │
      │                     ╱  Is change of  ╲                  │
      │                   ╱  applied voltage V  ╲    NO          │
      │                  ⟨ and electric conduction ⟩─────────────┘
      │                   ╲  time t instructed  ╱
      │                     ╲   by user?      ╱
      │                       ╲───────────╱
      │                           │ YES
      │                           ▼
      │           ┌──────────────────────────────┐
      │           │ Calculate, based on applied   │
      │           │ voltage V and/or electric     │  ⌇S506
      │           │ conduction time t, electric   │
      │           │ energy W to be supplied to    │
      │           │ heater                        │
      │           └───────────────┬──────────────┘
      │                           │           S508
      │                           ▼
      │                       ╱───────────╲
      │            NO       ╱               ╲      YES
      │        ┌──────────⟨   W > Wmax ?     ⟩──────────┐
      │        │            ╲               ╱           │
      │        │              ╲───────────╱             │
      │        ▼                                        ▼
      │  ┌──────────────────┐            ┌──────────────────────┐
      │  │ Drive heater by  │            │ Correct applied      │
      │  │ use of electric  │  ⌇S510     │ voltage V and/or     │  ⌇S512
      │  │ energy W, based  │            │ electric conduction  │
      │  │ on applied       │            │ time t, and drive    │
      │  │ voltage V and/or │            │ heater by use of     │
      │  │ electric         │            │ electric energy Wmax │
      │  │ conduction time  │            └──────────┬───────────┘
      │  │ t instructed by  │                       │
      │  │ user             │                       │
      │  └────────┬─────────┘                       │
      │           │                                 │
      └───────────┴─────────────────────────────────┘
```

S504: Is change of applied voltage V and electric conduction time t instructed by user?

S506: Calculate, based on applied voltage V and/or electric conduction time t, electric energy W to be supplied to heater

S508: $W > W_{max}$ ?

S510: Drive heater by use of electric energy W, based on applied voltage V and/or electric conduction time t instructed by user

S512: Correct applied voltage V and/or electric conduction time t, and drive heater by use of electric energy $W_{max}$

Fig. 5

Fig. 6A

Fig. 6B

EP 3 513 667 A1

Fig. 7

Fig. 8

```
                        ┌─────────┐
                        │  Start  │
                        └────┬────┘
                             │
                             ▼                    S802
                ◇─────────────────────────◇
               ╱  (Electric conduction time  ╲         NO
              ◇    of first atomizing unit) >   ◇──────────────┐
               ╲    (First cut-off time) ?     ╱               │
                ◇─────────────────────────◇                    ▼              S810
                             │                        ◇───────────────────◇
                            YES                      ╱   Is inhaling action  ╲      YES
                             │                      ◇    being continued?      ◇─────────┐
                             ▼                       ╲                        ╱          │
              ┌──────────────────────────┐            ◇───────────────────◇             │
              │  Stop electric conduction to │              │                            │
              │     first atomizing unit    │──S804         NO                           │
              └──────────────────────────┘              │                               │
                             │                           ▼                               │
                             │          ┌──────────────────────────────┐                │
                             │          │ Stop electric conduction to first  │           │
                             │          │ second atomizing units, and reset  │──S812     │
                             │          │    electric conduction time        │           │
                             │          └──────────────────────────────┘                │
                             │                                                           │
                             ▼                    S806                                   │
                ◇─────────────────────────◇                                             │
               ╱  (Electric conduction time  ╲         NO                               │
              ◇   of second atomizing unit) >  ◇──────────────┐                         │
               ╲   (Second cut-off time) ?    ╱               │                         │
                ◇─────────────────────────◇                    ▼              S814       │
                             │                        ◇───────────────────◇             │
                            YES                      ╱   Is inhaling action  ╲      YES  │
                             │                      ◇    being continued?      ◇─────────┘
                             ▼                       ╲                        ╱
              ┌──────────────────────────┐            ◇───────────────────◇
              │  Stop electric conduction to │              │
              │    second atomizing unit    │──S808         NO
              └──────────────────────────┘              │
                             │                           ▼
                             ▼          ┌──────────────────────────────┐
              ┌──────────────────────────┐│ Stop electric conduction to first  │
              │ Reset electric conduction time of││ second atomizing units, and reset │──S816
              │ first and second atomizing units │──S809│   electric conduction time        │
              └──────────────────────────┘└──────────────────────────────┘
```

Fig. 9

200

Processor 210

Memory 220

Communication interface 230

Display 240

User input device 250

Fig. 10

200

245

Output control

Total vapor

30%

246A

Flavor

30%

246B

Device status

Battery level    100%

248A

Cartomizer 1 level    100%

248B

Cartomizer 2 level    100%

248C

240

Fig. 11

**INTERNATIONAL SEARCH REPORT**

| International application No. |
| --- |
| PCT/JP2017/033889 |

A. CLASSIFICATION OF SUBJECT MATTER
Int.Cl. A24F47/00(2006.01)i

According to International Patent Classification (IPC) or to both national classification and IPC

B. FIELDS SEARCHED

Minimum documentation searched (classification system followed by classification symbols)
Int.Cl. A24F47/00

Documentation searched other than minimum documentation to the extent that such documents are included in the fields searched
Japanese Published Examined Utility Model Applications        1922-1996
Japanese Published Unexamined Utility Model Applications      1971-2017
Japanese Examined Utility Model Registrations                1996-2017
Japanese Registered Utility Model Specifications             1994-2017

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
| --- | --- | --- |
| Y<br><br>A | WO 2016/121143 A1 (JAPAN TOBACCO INC.) 04 August 2016, paragraphs [0033]-[0035], [0063]-[0068], fig. 1, 3 | 1-6, 16-17, 22, 24-25, 31-34<br>7-15, 18-21, 23, 26-30 |
| Y | WO 2014/115324 A1 (JAPAN TOBACCO INC.) 31 July 2014, paragraphs [0018]-[0024], fig. 1 | 1-6, 16-17, 22, 24-25, 31-34 |

☒ Further documents are listed in the continuation of Box C.          ☐ See patent family annex.

| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 21 November 2017 | 05 December 2017 |

| Name and mailing address of the ISA/ | Authorized officer |
| --- | --- |
| Japan Patent Office<br>3-4-3, Kasumigaseki, Chiyoda-ku,<br>Tokyo 100-8915, Japan | Telephone No. |

Form PCT/ISA/210 (second sheet) (January 2015)

## INTERNATIONAL SEARCH REPORT

| | International application No. |
|---|---|
| | PCT/JP2017/033889 |

C (Continuation).   DOCUMENTS CONSIDERED TO BE RELEVANT

| Category* | Citation of document, with indication, where appropriate, of the relevant passages | Relevant to claim No. |
|---|---|---|
| Y | US 2015/0245654 A1 (BEYOND TWENTY LTD.) 03 September 2015, paragraph [0485] | 5-6, 16-17, 22, 24-25, 31-34 |
| Y | WO 2015/046386 A1 (JAPAN TOBACCO INC.) 02 April 2015, paragraphs [0038], [0074] | 16-17, 22, 24-25, 31-34 |
| Y | WO 2016/135959 A1 (JAPAN TOBACCO INC.) 01 September 2016, paragraph [0062] | 16-17, 22, 24-25, 31-34 |

Form PCT/ISA/210 (continuation of second sheet) (January 2015)

**INTERNATIONAL SEARCH REPORT**
Information on patent family members

| International application No. |
| --- |
| PCT/JP2017/033889 |

| | | |
| --- | --- | --- |
| WO 2016/121143 A | 04 August 2016 | CN 107205482 A |
| WO 2014/115324 A1 | 31 July 2014 | Family: none |
| US 2015/0245654 A1 | 03 September 2015 | JP 2017-513513 A<br>WO 2015/128665 A1<br>EP 2964038 A<br>CN 106163307 A<br>KR 10-2016-0129024 A |
| WO 2016/135959 A1 | 01 September 2016 | CN 107249364 A<br>KR 10-2017-0106454 A |
| WO 2015/046386 A1 | 02 April 2015 | US 2016/0205998 A<br>EP 3039973 A<br>CN 105592737 A<br>KR 10-2016-0052607 A |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2015179388 A